# EUROPEAN PATENT SPECIFICATION

(11) **EP 4 176 256 B1**
(45) Date of publication and mention of the grant of the patent: **29.01.2025**
(21) Application number: 21742539.6
(22) Date of filing: 05.07.2021
(51) Int. Cl.: G01N 33/08, G01N 1/10

(54) **EGG DETERMINING METHOD AND DEVICE**
EIBESTIMMUNGSVERFAHREN UND -VORRICHTUNG
PROCÉDÉ ET DISPOSITIF DE DÉTERMINATION D'OEUF

(30) Priority: 05.07.2020 NL 2026004
(43) Date of publication of application: 10.05.2023
(73) Proprietor: In Ovo Holding B.V., 2332 KG Leiden (NL)
(72) Inventor: STUTTERHEIM, Wil Marijn, 2333 BA Leiden (NL); BRUINS, Wouter Sebastiaan, 2333 BA Leiden (NL); TEUNISSEN, Sebastiaan Frans, 2333 BA Leiden (NL); HANKEMEIER, Thomas, 2333 CC Leiden (NL); DROUIN, Nicolas Francois Pierre, 2333 CC Leiden (NL)
(74) Representative: HGF
(86) International application number: PCT/NL2021/050427
(87) International publication number: WO 2022/010348

(56) References cited:
- WO-A1-2021/145771
- WO-A2-02/083848
- WO-A2-2017/204636
- CN-A- 109 619 030
- US-A1- 2002 001 544
- US-A1- 2016 050 891
- US-A1- 2019 174 726
- US-A1- 2020 116 730

## Description

### Field of the invention

The present application provides a method and system for determining a characteristic of one or more eggs by automatically extracting a sample from one or more eggs and automatically analyzing an aliquot thereof in a mass spectrometer.

### Background of the invention

Discrimination between poultry eggs (hereinafter "eggs") on the basis of some observable quality is a well-known and long-used practice in the poultry industry. "Candling" is a common name for one such technique, a term which has its roots in the original practice of inspecting an egg using the light from a candle. Although eggshells appear opaque under most lighting conditions, eggs are actually somewhat translucent. Accordingly, when placed in front of a light, the contents of an egg can be observed.

In poultry hatcheries, one purpose of candling eggs is to identify and then segregate live eggs (i.e., eggs which are to be hatched to live poultry) from non-live eggs (e.g., clear eggs, dead eggs, rotted eggs, empty eggs, unfertilized eggs, etc.). For example, US4955728A and US4914672A describe a candling apparatus that uses infrared detectors and the infrared radiation emitted from an egg to identify live eggs. US4671652A describes a candling apparatus in which a plurality of light sources and corresponding light detectors are mounted in an array, and wherein eggs are passed between the light sources and the light detectors to identify live eggs.

In hatchery management, it may be desirable to separate birds based upon various characteristics, such as sex, diseases, genetic traits, etc. For example, it may be desirable to inoculate male birds with a particular vaccine and inoculate female birds with a different vaccine. Sex separation of birds at hatch may be important for other reasons as well. For example, turkeys are conventionally segregated by sex because of the difference in growth rate and nutritional requirements of male and female turkeys. In the layer or table egg industry, it is desirable to keep only females. In the broiler industry, it is desirable to segregate birds based on sex to gain feed efficiencies, improve processing uniformity, and reduce production costs.

Unfortunately, conventional methods of sexing birds may be expensive, labor intensive, time consuming, and typically require trained persons with specialized skills. Conventional methods of sexing birds include feather sexing, vent sexing, and DNA or blood sexing. About three thousand (3,000) chicks can be feather-sexed per hour at a cost of about 0.7 to 2.5 cents per chick. About fifteen hundred (1,500) chicks can be vent-sexed per hour at a cost of about 3.6 to 4.8 cents per chick. DNA or blood sexing is performed by analyzing a small sample of blood collected from a bird.

It would be desirable to identify the sex of birds, as well as other characteristics of birds, prior to hatching. Pre-hatch sex identification could reduce costs significantly for various members of the poultry industry. Although conventional candling techniques can discriminate somewhat effectively between live and non-live eggs, these conventional candling techniques may not be able to reliably determine sex and other characteristics of unhatched birds.

An alternative to conventional candling techniques is the analysis of the existence and the amounts of previously unidentified analytes for the determination of various characteristics of an egg, and the embryo therein. For example, WO2015179719A1 discloses an automated egg gender sorting system and method comprising a sampling module, including a candling system, a sampling system for extracting samples from an egg and depositing the samples on a liquid-immobilizing assay template, an assaying module that receives the sampled material, an egg treatment (vaccination) and gender sorting module, and optionally an egg holding system.

WO2019154493A1 discloses a method for sampling an egg, wherein the interior of the egg is fluidly coupled to a source of pressure, the pressure of the interior of the egg is controlled by the source of pressure, an amount of fluid, in particular allantoic fluid, is expelled from the interior of the egg to the exterior as a result of the pressure in the interior of the egg, and collecting a portion of the expelled fluid.

The aforementioned prior art does not disclose the use of a high accuracy system for analysis of the samples, such as mass spectrometry, let alone a system capable of applying industrial ratesMass spectrometry is a powerful analyte detection and measurement technique that has become the preferred method of detecting small molecule, amino acid, protein, peptide, nucleic acid, lipid, and carbohydrate analytes to a high accuracy for diagnostic purposes.

Mass spectrometry is currently not used in an industrial setting in the poultry industry to determine the sex or other characteristics of unhatched eggs. However, this method may enable the analysis of the existence and the amounts of previously unidentified analytes for the determination of various characteristics of an egg, and the embryo therein.

However, a lack of systems and methods for using mass spectrometry in an industrial setting in the poultry industry has thus far hindered the implementation of mass spectrometry in this field, in particular due to the relatively slow sample preparation, and sensitivity of the systems to pollution when in use outside a laboratory "clean room" setting.

The use of mass spectrometry for automated analysis of biological samples has been described previously. For example, WO2005009202A2 describes a method and system for automatic identification of bioagents via robotic DNA extraction, PCR followed by mass spectrometry but does not provide a means for taking and/or transferring samples from eggs.

EP3185016A1 describes a system layout for an automated system for biological sample preparation and analysis by mass spectrometry, but also does not provide a means for taking and/or transferring samples from eggs. The use of mass spectrometry in the non-invasive determination of the sex of an embryo of an egg has been described in for instance WO2014154513A1, which discloses a non-invasive method for determining the sex of an embryo of an egg by analyzing estradiol or testosterone levels via mass spectrometry. However, there is no information about a means for taking and/or transferring samples from eggs, let alone in an industrial setting.

WO2017204636A2 discloses a method and system for identifying a characteristic of an egg comprising obtaining a sample, preferably from the allantois, and preferably analysing the sample by mass spectrometry on certain biomarkers, preferably wherein a first biomarker comprises an amino compound having a molecular weight in the range of from 140 to 190 g/mole.

However, the prior art does not provide a device or method for identifying a characteristic of an egg comprising obtaining a sample, optionally processing the sample, and analyzing the sample by mass spectrometry in a manner with a sufficient speed and accuracy that is necessary for an industrial setting.Hence there remains a need for an automated device and method that provide a means for taking samples from eggs suitable for transferring the samples to a mass spectrometer for determination of one or more characteristics of the eggs in a speedy and economically viable manner. US2020/116730 A1 discloses determining egg characteristics using mass spectroscopy, but does not disclose ejecting an aliquot of a sample using sound or light energy. US2002/001544 A1 discloses atomising a sample for mass spectroscopy using acoustic waves, but not in the premises of egg analysis.

### Summary of the invention

The invention is set out in the appended set of claims. The following examples are for illustration purposes. Accordingly, in a first aspect, there is provided a method for determining a characteristic of one or more eggs, comprising the steps of: a.) automatically extracting one or more samples from one or more eggs; and b.) automatically analyzing the sample or an aliquot thereof in a mass spectrometer for the absence or presence and the amount of one or more molecules, to determine one or more characteristics of the egg.

In view of the above discussion, aspects of the present disclosure provide methods of processing eggs having an identified characteristic (e.g. sex) wherein material (e.g. allantoic fluid) is extracted from each of a plurality of eggs, the extracted material is assayed to identify eggs having a characteristic, and then eggs identified as having the characteristic are processed accordingly. For example, a method of processing eggs based upon sex, according to aspects of the present disclosure, includes extracting material from the eggs, assaying each live egg to identify the sex of each live egg. According to aspects of the present disclosure, an automated egg determining system is provided and includes four independent modules linked via a network.

In a further aspect, there is provided a system for determining eggs, comprising:
a. a conveyor system configured to transport one or more eggs to a sampling system;
b. a sampling system configured to extract a sample from one or more eggs, wherein the sampling system or a part thereof is movable towards a sample transfer system, or vice versa, to transfer the sample between the sampling system and the sample transfer system;
c. a sample transfer system configured to receive the sample for transfer to an assaying system; and
d. an assaying system comprising a mass spectrometer configured to receive the sample from the sample transfer system and to determine one or more characteristics of one or more eggs or the sample or an aliquot thereof.

There is provided a process of determining eggs comprising a system according to any of the preceding systems of the present disclosure.

Finally, there is provided the use of a system for automated determination of a characteristic in a multitude of eggs according to any of the preceding systems of the present disclosure.

### Short Description of the Figures

Figure 1 shows a flow chart for a system for taking samples from eggs, transferring the samples to an assaying system and determining one or more characteristics of the eggs.
Figure 2 shows a model of a device capable of holding one or more eggs, which is also capable of rotating the egg and/or positioning the egg relative to a candling unit.
Figure 3 shows a model for an apparatus containing several extractors, capable of moving the extractors from and to an instrument for holding comprising a multitude of sample containers.
Figure 4 shows a schematic overview of different steps of a system for taking one or more samples from one or more eggs.
Figure 5 shows a detailed schematic cross section of an extractor positioned in a (chicken) egg at the moment before taking a sample.
Figure 6 shows a schematic cross section of a hollow elongated object of an extractor positioned in an egg.
Figure 7 shows a schematic overview of the egg and a spacer system during candling.
Figure 8 shows a schematic of the egg in contact with a spacer object and out of contact with flat springs prior to being rotated.
Figure 9 shows a comparison of signal intensities during mass spectrometry analysis of different solvent compositions of the gas or liquid stream according to the invention wherein the one or more molecules is Biomarker 1.
Figure 10 shows the influence of sample dilution and the amount of formic acid added to the sample on the signal intensity during mass spectrometry analysis.
Figure 11 shows three different methods for determining the sex of the embryo of the egg using the method of the invention.

### Detailed description

Biomarker herein is understood to mean a substance that can indicate alone or in combination with other substances a biological state or condition.

Veterinary drug herein is understood to mean a substance or a combination of substances to treat, prevent or diagnose disease in animals.

Pesticide herein is understood to mean a substance that can control pests. Pests comprise plants, arthropods, mollusks, nematodes, fungi, bacteria, viruses and other microbes that cause damage to objects, spread disease or are disease vectors. Pesticides control pests by killing, repelling or reducing the growth and/or reproduction of pests.

Candling is a common method used in embryology to study the growth and development of an embryo inside an egg. The term "candling" is understood herein to mean using a light source of sufficient strength directed at an egg enabling the detection of any structures inside an egg, preferably at minimum the air cell. A candling unit typically is a system comprising one or more light sources and one or more detectors.

Cleaning herein is understood to mean removing undesired substances, such as dirt, infectious agents, and other impurities, from an object or environment. Cleaning can be achieved by a variety of different means, for example by flushing with water or a solution containing a soap or detergent, using sound waves to shake particulates loose, using steam cleaning, applying one or more disinfectants, subjecting the object or environment to a sufficiently high temperature to kill or otherwise inactivate infectious agents or by thermal cleaning; a combined process involving pyrolysis and oxidation.

Characteristics of an egg are understood to mean either characteristics of the egg itself or characteristics of the embryo inside the egg. Examples of such characteristics are sex, feeding state, health status or developmental status. The feeding state is the amount and quality of beneficial nutrients inside the embryo or the egg or the ratio between both. The health status is the degree of the state of physical, anatomic, physiological well-being in which disease and infirmity are absent. The developmental status is the degree of development of the biological, biochemical and physical features of the embryo.

In the context of the present invention, the term solvent is a material capable of dissolving or dispersing or emulsifying another material. A solvent may be used in the context of the sample prior to contact with the gas or liquid stream or in context of the gas or liquid stream itself. A person skilled in the art understands that a solvent used in preparing the sample may be different than the solvent used in the gas or liquid stream that is used for entraining and/or transporting a sample or an aliquot to a mass spectrometer, because the solvent needs to fulfull different requirements for suitable use in each case.

Contacting the sample with other materials is meant to be understood as adding the sample to other materials, including other materials in solvents or emulsions, or vice versa. The adding can be done in tubes, reaction tubes, instruments for holding comprising one or more sample containers such as microplates, or other containers commonly used in a laboratory for handling or preparation of samples. During or after adding the sample or the other materials to each other they can be blended, mixed and/or incubated in order to optionally react the sample or an analyte therein and one or more of the other materials with each other. At this stage the sample can be subjected to methods for purification, concentration and/or isolation in order to separate, purify, concentrate and/or isolate materials from the sample.

Other materials are meant to be understood to be materials, in particular a fluid or a material in solution, used for further processing of the sample in order for one or more characteristics of the egg or the embryo to be assessed. Other materials comprise reference material, a diluent, a solvent, an enzyme, a binding agent or other material reacting with or binding to an analyte in the sample.

Reference material advantageously comprises a material, sufficiently homogeneous and stable with respect to one or more specified properties, which has been established to be fit for its intended use in a measurement process.

A conveyor is a commonly known piece of mechanical handling equipment that moves materials from one location to another. A conveyor system in the egg industry typically transports eggs from the hens throughout a facility, from any of to any of: collection, grading, incubating, hatching, sorting, packing or shipping. Eggs are commonly placed in trays or flats or can be placed directly on the conveyor. Conventional incubating or setting trays include the Chick Master^{®} 54 tray, the Jamesway^{®} 42 tray, and the Jamesway^{®} 84 tray (in each case, the number indicates the number of eggs carried by the tray). There are some incubating trays, such as the La Nationale^{®} incubating tray, which are sufficiently large enough to include an even higher total number of eggs, such as 132. Eggs can also be transported via a conveyor in a variety of other ways such as for example by a single carrier or a group of carriers comprising an egg accommodation and an egg clamping system as described in WO2019096372A1. A conveyor can be spacially configured in a variety of manners such as under a vertical angle or in a circular or semicircular configuration or a combination thereof.

A tray herein is understood to mean a type of product created and designed in various colors, materials, mechanisms, shapes, sizes and styles used to hold and protect a specific number of eggs.

A detector is herein understood to mean a device or instrument designed to detect the presence of structures inside an egg or alternatively light or sound waves passing through or reflecting from the inside of the egg. The waves comprise light, other electromagnetic radiation or ultrasound sound waves. The detector comprises a sensor that detects and conveys information used to make an image. Two main types of electronic image sensors are the charge-coupled device (CCD) and the active-pixel sensor (CMOS sensor). Alternatively, a Quanta Image Sensor can be used.

The central egg axis is also commonly known as the major axis of the egg. The major axis spans the greatest possible distance between the tip of the pointed side of the egg and the base of the blunt side of the egg.

Herein, sex and gender are used interchangeably and are understood to mean the biological sex of an organism, i.e. male or female.

Fluid connection is understood herein as to be a connection between two or more systems comprising gas and/or fluid as a means of transporting substances between the two or more systems.

Mass spectrometry is a generic method that allows detection of a large variety of different small molecule metabolites. Ion spray and electrospray mass spectrometry have been used in many different fields for the analysis of organic compounds and for characterization of biomacromolecules. For a general discussion of mass spectrometry theory and techniques, see, e.g., Kirk-Othmer Encyclopedia of Chemical Technology, Volume 15, Fourth Edition, pages 1071-1094, and all references therein. See, also, Mass Spectrometry for Biotechnology, G Siuzdak, Academic Press, San Diego, Calif., 1996; Electrospray Ionization Mass Spectrometry: Fundamentals, Instrumentation, and Applications, R. Cole (Ed.), Wiley and Sons, 1997; Mass Spectrometry for Chemists and Biochemists, Johnstone et al., Cambridge University Press, 1996; Mass Spectrometry: Principles and Applications, Hoffman et al., Wiley and Sons, 1996; Quadrupole Mass Spectrometry and its Applications, Dawson (ed.), Springer Verlag, 1995; and Advances in Mass Spectrometry, Karjalainen et al. (eds.), Elsevier Science, 1998).

Mass Spectrometers are usually coupled to a separation technique, such as high-performance liquid chromatography or capillary zone electrophoresis, which is performed in-line with the mass spectrometry analysis. However, this usually slows down the rate of mass spectrometry and hence limits its use as a high-throughput method. Also, it may make the method and system vulnerable to use in an industrial setting, e.g. a poultry farm.

Electrospray methods are used instead of gas chromatography procedures because no prior derivatization is required to inject the sample. Flow injection analysis methods (FIA) with ion spray-ionization and tandem mass spectrometry further the ability of the present invention to perform high-throughput mass spectrometry analysis. The ion spray method allows the samples to be injected without prior derivatization and the tandem mass spectrometry (MS-MS) allows extremely high efficiency in the analysis. Therefore, no column separation is needed. Electrospray ionization is a very mild ionization method that allows detection of molecules that are polar and large which are typically difficult to detect in GC-MS without prior derivatization. Modern electrospray mass spectrometers detect samples in femtomole quantities. In the present method, sample aliquots are injected in nanomolar concentrations, attomolar concentrations or lower, such that quantitation remains reproducible with a standard error ranging from 0.5%-1 % at most.

Electrospray ionization (ESI) is a technique used in mass spectrometry to produce ions using an electrospray in which a high voltage is applied to a liquid to create an aerosol. It is especially useful in producing ions from macromolecules because it overcomes the propensity of these molecules to fragment when ionized. ESI is different from other ionization processes (e.g. matrix-assisted laser desorption/ionization (MALDI)) since it may produce multiple-charged ions, effectively extending the mass range of the analyser to accommodate the kDa-MDa orders of magnitude observed in proteins and their associated polypeptide fragments. ESI is a so-called 'soft ionization' technique, since there is very little fragmentation. This can be advantageous in the sense that the molecular ion (or more accurately a pseudo molecular ion) is always observed, however very little structural information can be gained from the simple mass spectrum obtained. Another important advantage of ESI is that solution-phase information can be retained into the gas-phase.

Matrix-assisted laser desorption/ionization (MALDI) is an ionization technique that uses a laser energy absorbing matrix to create ions from large molecules with minimal fragmentation. It is similar in character to electrospray ionization (ESI) in that both techniques are relatively soft (low fragmentation) ways of obtaining ions of large molecules in the gas phase, though MALDI typically produces far fewer multi-charged ions. MALDI methodology is a three-step process. First, the sample is mixed with a suitable matrix material and applied to a metal plate. Second, a pulsed laser irradiates the sample, triggering ablation and desorption of the sample and matrix material. Finally, the analyte molecules are ionized by being protonated or deprotonated in the hot plume of ablated gases, and then they can be accelerated into whichever mass spectrometer is used to analyze them.

Atmospheric-pressure chemical ionization (APCI) is an ionization method used in mass spectrometry which utilizes gas-phase ion-molecule reactions at atmospheric pressure (105 Pa), commonly coupled with high-performance liquid chromatography (HPLC). APCI is a soft ionization method similar to chemical ionization where primary ions are produced on a solvent spray. The main usage of APCI is for polar and relatively less polar thermally stable compounds with molecular weight less than 1500 Da.

Any of the aforementioned mass spectrometry techniques, and other such as such as desorption electrospray ionization (DESI) and laser diode thermal desorption (LDTD), may be used.

Other techniques of use include, but are not limited to, neutral loss and parent ion scanning. Neutral loss is a method of mass spectrometry scanning in which all compounds that lose a neutral molecular fragment, i.e., a specific neutral fragment, during collision induced dissociation (CID) are detected. Parent ion mode detects all compounds that produce a common daughter ion fragment during CID. These techniques are optionally used, e.g., to quantitate the amount of product and starting material simultaneously. For systems in which the expected product is not known, e.g., a standard is not available, the neutral loss and/or parent ion method allows backtracking or deconvolution based on fragmentation patterns to determine the structure and/or identity of the starting material. For example, the parent mass is determined based on the various fragments produced.

Segmented flow or flow injection is understood to mean an approach to chemical analysis accomplished by injecting a plug of sample into a flowing carrier (gas or liquid) stream. The carrier solution and sample then meet at mixing points with reagents and react. The reaction product then flows through a detector. In addition, air can be optionally injected into the sample or reagent streams.

Egg herein is understood to mean a bird egg of a domesticated bird kept by humans for its eggs, meat or feathers. These birds are typically members of the superorder Galloanserae, but can also include for example ostriches, pigeons or doves. The eggs may also be employed to produce vaccines.

Extracting a sample from an egg is herein understood to mean extracting a sample from any part of the entirety of the egg and its contents at any stage of the egg. The parts include the embryo, the unhatched chick, the shell, all compartments, membranes and gaseous, fluid and solid components.

An example is provided of a method for determining a characteristic of one or more eggs in an automated manner, comprising the steps of:
a. extracting a sample from one or more eggs in an automated manner; and
b. analyzing the sample or an aliquot thereof in an automated manner in a mass spectrometer for the absence or presence and the amount of one or more molecules, to determine one or more characteristics of the egg.

It was found that a particular further instance of the method advantageously permitted to increase the amount of samples that can be transferred to a mass spectrometer per time unit, while also enabling the analysis of the samples with a high accuracy with regard to the absence or presence and the amount of one or more molecules characteristic of the egg in order to determine one or more characteristics of the egg. This further instance is set out in more detail in the following section.

### Aliquot ejection from the sample and subsequent entrainment

Preferably, step a. further comprises the following steps:
i. holding and transferring the sample through one or more instruments to a unit for holding a multitude of sample containers;
ii. ejecting an aliquot from the sample by applying energy to at least an amount of the sample;
iii. entraining the ejected aliquot in a carrier comprising a gas or liquid stream; and
iv. transporting the ejected aliquot to a mass spectrometer using the gas or liquid stream.

Preferably, the energy is supplied by sound or light energy. Preferably, the energy is light energy, more preferably laser energy. More preferably, the energy comprises a soundwave, preferably a multitude of soundwaves, preferably a soundwave or a multitude of soundwaves of sufficient energy. Even more preferably, the energy comprises a sufficient pulse rate enabling the ejecting an aliquot of a sufficient size. Preferably, the sound energy or soundwave comprises a frequency of from 300 to 2000 Hz, preferably of from 600 to 1000 Hz, more preferably of from 700 to 900 Hz or about 800 Hz.

The inventors have found that by ejecting an aliquot from the sample by applying energy to at least an amount of the sample and subsequently directly entraining the ejected aliquot in a carrier comprising a gas or liquid stream, thereby not using a conventional injector system such as conventional liquid chromatography injectors, there is no carryover of aliquots resulting from injection valve or needle contamination. Moreover, by bypassing the conventional method of injecting samples for analysis by mass spectrometry as such, the samples can be transferred to the mass spectrometer at a higher speed, thereby enabling the use of mass spectrometry analysis according to the invention in an industrial setting. At the same time, a high accuracy mass spectrometry analysis with regard to the absence or presence and the amount of one or more molecules characteristic of the egg can be achieved.

"Sound energy" herein is also understood to mean acoustic energy. Sound energy is preferably applied to the sample via an acoustic transducer. The acoustic transducer converts mechanical or electrical energy into sound energy. Preferably, the acoustic transducer controls the energy, preferably by being configured to direct a multitude of soundwaves to a focal point or area located on the surface of the sample, preferably in such a way that an aliquot of predetermined or sufficient size is ejected from the sample.

Applying sound energy to at least an amount of the sample comprises controlling the energy, preferably in such a way that an aliquot of sufficient size is ejected from the sample. Preferably, applying sound energy comprises applying sound energy originating from an area opposite the area where the aliquot will be entrained. Applying sound energy to the sample then ejects an aliquot from the surface of the sample towards the area where the aliquot will be entrained. As such, a discrete aliquot, preferably comprising a multitude of droplets, for example in the form of a mist, can be created next to the surface area of the sample.

Laser ablation or photoablation is a process wherein a laser is used to remove or ablate material from the surface of the material. The energy provided by the laser to the surface of the material causes part of the material to evaporate or sublimate. Preferably, the laser energy according to the invention is provided by a pulsed laser or a continuous wave laser, more preferably a pulsed laser. An excimer or exciplex laser is a particular preferred laser that preferably emits ultraviolet light energy with a wavelength of from 100 to 400 nm. The degree of ablation can be controlled by for example adjusting the laser wavelength and pulse length in relation to the optical properties of the material. According to the invention, applying laser energy to at least an amount of the sample comprises controlling the energy, preferably in such a way that an aliquot of predetermined or sufficient size is ejected from the sample. As such a discrete aliquot, preferably comprising a multitude of droplets, for example in the form of a mist, can be created next to the contact area of the laser and the surface area of the sample.

The aliquot according to the invention preferably has an average volume of at least 2 nl, preferably of at least 3, 4 or 5 nl, or of from 2 to 10 nl, preferably of from 2, 3 or 4 to 7, 8 or 9 nl. The aliquot according to the invention preferably comprises a multitude of droplets. Preferably, the multitude of droplets comprise individual droplets, preferably wherein the individual droplets have an average volume of at least 2 nl, preferably of at least 3, 4 or 5 nl, or of from 2 to 10 nl, preferably of from 2, 3 or 4 to 7, 8 or 9 nl. It was found that a minimum volume was needed to provide a sufficient amount of the one or more molecules in order to determine one or more characteristics of the egg. Higher volumes provide a progressively smaller contribution to the ability of the mass spectrometer to analyse the amount of the one or more molecules due to the matrix effect.

An aliquot or a sufficient amount of the aliquot ejected by sound or laser energy merges into the carrier comprising a gas or liquid stream by contacting the carrier or gas or liquid stream. The amount of aliquot contacted can be increased by adjusting the direction of the energy that is applied to the sample for ejection of the aliquot. The amount can for example also be increased by adjusting the distance between the sample and the stream. Preferably, the carrier according to the invention comprises a solvent. Preferably, the gas or liquid stream according to the invention comprises a solvent.

Entraining the ejected aliquot in a carrier comprising a gas or liquid stream preferably comprises entraining the aliquot into a hanging droplet or a continuous stream, preferably a continuous stream, preferably comprising a solvent. By employing a continuous stream carryover between aliquots of different samples can be minimized, which contributes to the accuracy of the analysis.

Preferably, transporting the ejected aliquot to a mass spectrometer using the gas or liquid stream comprises a continuous stream, continuous infusion, segmented flow and/or flow injection. This enables a speedy, automated and segmented in-line transfer and/or reaction of aliquots prior to analysis. Continuous infusion is understood to mean a continuous infusion of the stream, preferably comprising the aliquots, into the mass spectrometer for analysis.

According to an example, before and/or during step ii. of ejecting the aliquot, a step of ionizing the sample can advantageously be employed. Preferably, ionizing the sample comprises applying a high voltage above the sample, thereby generating an ionized sample. Preferably, the voltage is of from 500 to 5000 V.

Preferably, the method comprises prior to step a. the step of: providing a system that is configured to introduce a sample or an aliquot thereof to a mass spectrometer comprising a sampling probe having an open end presenting an air interface for receiving the sample, an inlet connected to the sampling probe supplying a gas or liquid stream capable of entraining the sample, an outlet connected to the sampling probe receiving the gas or liquid stream, wherein the outlet is configured to be in fluid communication with a mass spectrometer. Preferably, the gas or liquid stream is comprised by the carrier. An aliquot is advantageously ejected and directed from the sample towards an opening of the mass spectrometer (e.g. an open port), wherein the aliquot is immediately entrained in a gas or liquid stream comprising a solvent. The aliquot may then be transported and processed according to conventional mass spectrometry methods.Prefaebly, this may involve transferring the sample into a sample container, or alternatively, when using an in-line system, a

An example of a commercial system employing a system comprising a sampling probe having an open end as described above (also termed "open port interface") coupled with an acoustic (i.e. sonic) aliquot ejection system (also termed "acoustic droplet ejection") and a mass spectrometer is the Echo^{®} MS system manufactured by SCIEX, which is a preferred system to execute the method according to the invention. The Echo^{®} MS system can reach an analysis speed of up to 3 samples per second, which is more than sufficient for an industrial setting and is higher than what normally is achieved by conventional mass spectrometry analysis.

Ejecting an aliquot from the sample by applying energy to at least an amount of the sample and subsequently entraining the ejected aliquot in a carrier comprising a gas or liquid stream or in a gas or liquid stream enables the ejection of nanoliter-scale droplets into the carrier or gas or liquid stream, where the droplets are diluted to an extent that background interference from abundant (bio)molecules in the sample is no longer an issue. Because of the dilution, complex biological samples can be introduced without additional preparation. Combined with the design of the open port interface, the likelihood of a carryover signal is also reduced. This ultimately results in a more efficient process enabling a high-throughput sample analysis.

The inlet and the outlet according to the invention can be positioned relative to each other in various configurations known in the art. For example, the inlet and the outlet can both be a tube, wherein the the inlet surrounds the outlet at their connection to the sampling probe. The gas or liquid stream may be configured in such a way that the rate of the supply via the inlet and the rate of removal via the oulet differ from each other, thereby generating a gas or liquid stream that is in a vortex or meniscus configuration at the open end. To this end, preferably, the rate of supply is greater than the rate of removal, thereby generating a gas or liquid stream that is in a meniscus configuration, preferably a convex meniscus. More preferably, the rate of removal is greater than the rate of supply, thereby generating a gas or liquid stream that is in a vortex configuration, preferably a critical or supercritical vortex configuration.

In order to achieve a flow rate of the gas or liquid stream and subsequent aliquot analysis throughput that is at a rate sufficiently high for industrial applications using a sampling probe having an open end presenting an air interface for receiving the sample according to the invention, it is advantageous to reduce any negative capillary and Venturi effects that are generated by the gas or liquid stream. This can be achieved by a person skilled in the art by for example varying the respective diameters, lengths and/or materials of the transporting means of the inlet and/or outlet or by adjusting, preferably increasing, the pressure of the gas or liquid streams.

In order to improve the flow rate of the gas or liquid stream, according to the invention, the gas or liquid stream, preferably the solvent, preferably has a low viscosity. Preferably, the solvent comprises, substantially consists of or is methanol. Preferably, the gas or liquid stream comprising a solvent does not comprise formic acid. Methanol as the sole solvent in the gas or liquid stream was found to be very suitable for detection of the one or more molecules by the mass spectrometer as the background signal was very low. Addition of formic acid to the gas or liquid stream increased the background signal. According to the invention, preferably, the gas or liquid stream consists of methanol, preferably wherein the one or more molecules comprises or is Biomarker 1.

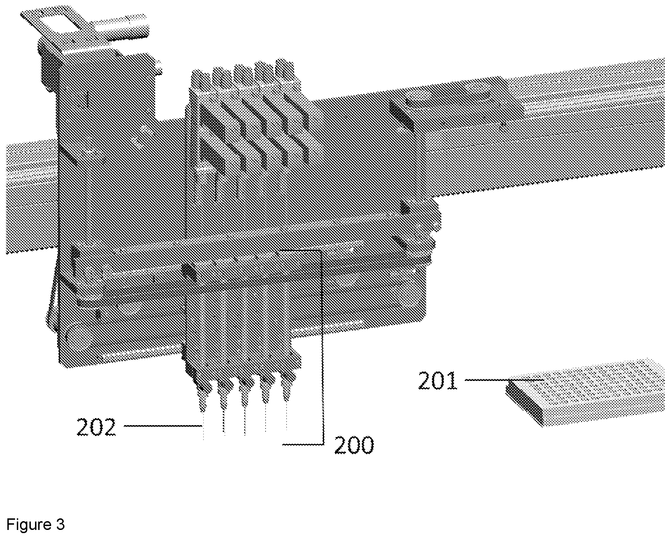

Extracting a sample from one or more eggs in an automated manner and subsequently holding and transferring the sample through one or more instruments to a unit for holding a multitude of sample containers or to a unit for transferring and handling a multitude of samples in line, wherein the one or more instruments preferably comprise the means of extracting or a means of in-line transferring the sample from the means of extracting. An example of the situation of the one or more instruments comprising the means of extracting is described in figure 3. Alternatively, the one or more instruments comprising a means of in-line transferring the sample from the means of extracting comprises a system where the means of extracting are not moved towards the unit for holding or vice versa, but wherein the extracted sample is transferred in-line via one or more tubes to the unit for holding.

Preferably, ejecting an aliquot from the sample by applying energy to at least an amount of the sample comprises the sample having a liquid-air interface surface plane that is suitably angled towards the carrier comprising a gas or liquid stream or the gas or liquid stream in order for a sufficient or maximum amount of ejected aliquot to contact the carrier or gas or liquid stream.

Preferably, the sample has a substantially horizontal or concave liquid-air interface surface area, preferably horizontal.The liquid-air interface surface area is also known as a meniscus, which is formed due to capillary action; interactions between the sample and the (wall of the) container and interactions between the components of the sample. In practice, the inventors have found that a substantially horizontal or concave liquid-air interface surface area of the sample, preferably horizontal, enables the ejection of an aliquot by applying energy, preferably sound energy, according to the invention of the highest volume and at the most ideal direction towards the carrier comprising a gas or liquid stream, which is preferably accessed via an open port interface, while also obtaining a high level of consistency between the different ejected aliquots.

Prior to ejecting an aliquot, the method preferably comprises a step of removing an amount of sample from a sample container that enables the formation of a substantially horizontal or concave liquid-air interface surface area of the sample. The amount of sample depends on the composition of the sample and the container and the dimensions of the container. For example, in a well of a 384-well plate suitable for ejection of an aliquot by sonic energy, containing 30 µl of sample, 10 µl of the sample can be removed to obtain a wall wetting on a sufficiently large surface to obtain a suitably centered and horizontal meniscus for improved ejection according to the invention. Alternatively, a dedicated injector may be employed that does not require the presence of a well plate.

As such, this is an improvement over conventional methods of obtaining a substantially horizontal or concave liquid-air interface surface area of a sample that comprise centrifugation and orbital shaking of the sample. Centrifugation and orbital shaking are time-consuming and laborious and are difficult and expensive to automate. By merely removing or aspirating a sufficient amount of sample prior to ejecting the sample can be prepared to a highly satisfactory degree for efficient ejection while also maintaining consistency and throughput speed. In addition, the removal of the amount of sample has the advantage of removing air bubbles from the sample that would also interfere with a proper ejection, consistency and/or analysis of the sample. Conventional means for removing air bubbles from samples comprise centrifugation and orbital shaking of the sample.

Prior to ejecting the aliquot, the method preferably comprises a step of diluting the sample, preferably using a suitable buffer, suitable diluent or water, more preferably water. Preferably, diluting the sample comprises using a solution comprising formic acid and preferably water. Preferably, the solution comprising formic acid comprises of from 0.1 to 20% formic acid (w/w), preferably of from 0.1 to 10%, more preferably of from 0.1 to 4%, even more preferably of from 0.5 to 2% or about 1% formic acid (w/w). Preferably, the suitable buffer, suitable diluent or water has a minimal effect on the ionization potential of the sample for subsequent analysis in a mass spectrometer, while also having a minimal effect on the stability of the one or more molecules.

Prior to ejecting the aliquot, the method preferably comprises a step of contacting the sample or an aliquot thereof with an amount of reference material, preferably wherein the amount is known. This to enable the determination of the amount and/or absence or presence of one or more analytes for determination of one or more characteristics of the sample and by extension the egg or the embryo inside the egg. However, the use of a standard may not be necessary once a system is well calibrated.

The one or more molecules preferably comprises 3-[(2-aminoethyl)sulfanyl]butanoic acid, also known as Biomarker 1. When the one or more molecules comprise Biomarker 1, the reference material preferably is suitably Biomarker 1-d4, i.e. 3-[(2-amino(2D₄)ethyl)sulfanyl] butanoic acid, which is a deuterated internal standard.

Using this approach, it is possible to compensate for batch to batch variation of the matrix effect, as well as injection variability and long-term source fouling.

Sampling of the egg: Preferably, step a. comprises determining the location of the air cell, the allantois and/or a blood vessel of the egg. Preferably, step a. further comprises determining the location of a preferred sample extraction point in the egg or at the exterior surface of the egg and collecting the sample or an aliquot thereof at the preferred sample extraction point.

Preferably, the one or more molecules comprise a biomarker, a veterinary drug or a pesticide. More preferably, the one or more molecules comprise a biomarker.

Preferably, the sample comprises an allantoic fluid sample or a blood sample, more preferably an allantoic fluid sample.

Preferably, extracting the sample comprises expelling the sample from the interior of the egg to the exterior of the egg as a result of controlling pressure in the interior of the egg and collecting the sample or an aliquot thereof at the exterior surface or interior-exterior intersection of the egg.

Preferably, controlling pressure comprises engaging a contact area of the egg shell, preferably at the air cell.

An embodiment of the invention relates to a method for determining a characteristic of one or more eggs, comprising the steps of:
a. automatically extracting a sample from one or more eggs; and
b. automatically analyzing the sample or an aliquot thereof in a mass spectrometer for the absence or presence and the amount of one or more molecules, to determine one or more characteristics of the egg.

Step a. further comprises the following steps:
i. holding and transferring the sample through one or more instruments to a unit for holding a multitude of sample containers or a unit for transferring a multitude of samples in-line;
ii. ejecting an aliquot from the sample, by applying sound or light energy;
iii. entraining the ejected aliquot in a carrier comprising a gas or liquid stream; and
iv. transporting the ejected aliquot to a mass spectrometer using the gas or liquid stream.

Preferably, the method comprises prior to step a. the step of: providing a system that is configured to introduce a sample or an aliquot thereof to a mass spectrometer comprising a sampling probe having an open end presenting an air interface for receiving the sample, an inlet connected to the sampling probe supplying a gas or liquid stream capable of entraining the sample, an outlet connected to the sampling probe receiving the gas or liquid stream, wherein the outlet is configured to be in fluid communication with a mass spectrometer.

An aliquot is preferably ejected and directed from the sample towards an opening of the mass spectrometer, e.g. an open port, wherein the aliquot is immediately entrained in a gas or liquid stream, preferably also comprising a carrier, e.g. a solvent. The aliquot is then transported and processed according to conventional mass spectrometry methods.

Preferably, step a. comprises determining the location of the air cell, the allantois and/or a blood vessel of the egg.

Preferably, step a. further comprises determining the location of a preferred sample extraction point in the egg or at the exterior surface of the egg and collecting the sample or an aliquot thereof at the preferred sample extraction point.

Preferably, the one or more molecules comprise a biomarker, a veterinary drug or a pesticide. More preferably, the one or more molecules comprise a biomarker.

Preferably, the sample comprises an allantoic fluid sample or a blood sample.

Preferably, extracting the sample comprises expelling the sample from the interior of the egg to the exterior of the egg as a result of controlling pressure in the interior of the egg and collecting the sample or an aliquot thereof at the exterior surface or interior-exterior intersection of the egg.

Preferably, controlling pressure comprises engaging a contact area of the egg shell, preferably at the air cell.

System for determining a characteristic of one or more eggs: There is provided an automated egg determining system, comprising:
a. a conveyor system configured to transport one or more eggs to a sampling system;
b. a sampling system configured to extract a sample from one or more eggs;
c. a sample transfer system configured to receive the sample for transfer to an assaying system; and
d. an assaying system comprising a mass spectrometer configured to receive the sample from the sample transfer system and to determine one or more characteristics of one or more eggs or the sample or an aliquot thereof.

Preferably, the automated egg determining system comprises a sampling system wherein the sampling system or a part thereof is movable towards a sample transfer system, or vice versa, to transfer the sample between the sampling system and the sample transfer system.

Various preferred non-limitative implementations of the conveyor system are foreseen. Preferably, the conveyor system is configured to transport the egg in a direction at an angle between the central egg axis and a horizontal plane in the range of from 20 to 80 degrees. Preferably, the conveyor system is configured to transport the egg in a linear and/or radial trajectory.

Preferably, the conveyor system comprises an egg manipulator configured to move the egg into a sampling position for extracting a sample from the egg.

Preferably, the egg manipulator comprises a mechanical or air pressure position maintaining and holding unit to hold, move and/or lift the egg. This unit is intended to hold and maintain the egg in a fixed position by mechanical means or by means of air pressure.

Preferably, the egg manipulator is configured to hold and release the egg via a mechanical means comprising movable objects configured to move into or out of contact with the egg or via a vacuum means.

Preferably, the egg manipulator is configured to rotate the egg.

Various preferred non-limitative implementations of the sampling system are foreseen.

Preferably, the sampling system comprises an egg manipulator configured to move the egg into a sampling position for extracting a sample from the egg.

Preferably, the sampling system comprises a means to determine the location of the air cell or allantois of the egg.

Preferably, the sampling system comprises one or more multi-axis robot arms comprising one or more extractors.

Preferably, the sampling system comprises a candling unit comprising one or more light sources and one or more detectors.

Preferably, the sampling system comprises a system to control air pressure inside the egg and/or to apply a vacuum to the inside of the egg.

Preferably, the sampling system is movable in at least a direction parallel to the conveyor system.

Preferably, the sampling system is configured to determine a sampling position based on one or more egg parameters, wherein the egg parameters are length of the egg, weight of the egg, color of the egg, image of the egg, dimension of the egg, circumference of the egg, incubation time of the egg, and/or age of the parent stock of the egg.

Preferably, the sampling system is in a fluid connection with the sample transfer system.

Various preferred non-limitative implementations of the sample transfer system are foreseen.

Preferably, the sample transfer system is configured to hold and transfer the sample via one or more instruments for holding comprising a multitude of sample containers or via one or more instruments for holding a multitude of sample containers, preferably wherein the sample transfer system is configured to receive the sample via a fluid connection from the sampling system and wherein the sample transfer system is configured to transfer the sample to the one or more instruments for holding, preferably wherein the fluid connection comprises an in-line means of transferring the sample.

Preferably, the sample transfer system is configured to receive the sample via a fluid connection from the sampling system, preferably wherein the fluid connection comprises an in-line means of transferring the sample. It was found that a particular further improvement of the method according to the invention increases the amount of samples that can be transferred to the mass spectrometer per time unit, while also enabling the analysis of the samples with a high accuracy with regard to the absence or presence and the amount of one or more molecules characteristic of the egg in order to determine one or more characteristics of the egg. In order to implement this further improvement the following system according to the invention is foreseen.

Preferably, the sample transfer system comprises a system that is configured to transfer a sample or an aliquot thereof according to the following steps:
a. an aliquot from the sample is ejected by applying energy to an amount of the sample;
b. the ejected aliquot is entrained in a carrier comprising a gas or liquid stream; and
c. the entrained aliquot is transported into the mass spectrometer using the gas or liquid stream.

Preferably, the energy is sound or light energy. Preferably, the energy is light energy, more preferably laser energy. More preferably, the energy is sound energy. More preferably, the energy comprises a soundwave, preferably a multitude of soundwaves, preferably a soundwave or a multitude of soundwaves of sufficient energy.

Preferably, the sample transfer system comprises an acoustic transducer. Preferably, the acoustic transducer is configured to apply energy to an amount of the sample. Preferably, the acoustic transducer is configured to control the energy, preferably configured to direct a multitude of soundwaves to a focal point or area located on the surface of the sample, preferably in such a way that an aliquot of predetermined or sufficient size is ejected from the sample. Preferably, the acoustic transducer is configured to apply energy via pulses, thereby enabling the ejection of a discrete amount of aliquot. Preferably, the acoustic transducer is configured to apply energy with a frequency of from 300 to 2000 Hz, preferably of from 600 to 1000 Hz, more preferably of from 700 to 900 Hz or about 800 Hz. Preferably, the acoustic transducer is configured to apply sound energy to an amount of the sample, wherein the energy originates from an area opposite to the area where the aliquot will be entrained, and preferably further in line with the amount of the sample and the area where the aliquot will be entrained. Preferably, the acoustic transducer is configured to apply sound energy to at least an amount of the sample by controlling the energy, preferably in such a way that an aliquot of sufficient size is ejected from the sample.

Preferably, the sample transfer system comprises a laser, preferably a pulsed laser or a continuous wave laser, more preferably a pulsed laser, even more preferably an excimer or exciplex laser. Preferably, the laser is configured to apply laser energy having a wavelength of from 100 to 400 nm. Preferably, the laser is configured to apply laser energy to at least an amount of the sample comprises by controlling the energy, preferably in such a way that an aliquot of predetermined or sufficient size is ejected from the sample.

Preferably, the sample transfer system is configured to eject an aliquot having an average volume of at least 2 nl, preferably of at least 3, 4 or 5 nl, or of from 2 to 10 nl, preferably of from 2, 3 or 4 to 7, 8 or 9 nl.

Preferably, the carrier comprises a solvent. Preferably, the gas or liquid stream according to the invention comprises a solvent.

The sample transfer system is configured to entrain the ejected aliquot in a carrier comprising a gas or liquid stream, wherein preferably the system is configured to entrain the aliquot into a hanging droplet or a continuous stream, preferably a continuous stream, preferably comprising a solvent.

Preferably, the sample transfer system is configured to transport the ejected aliquot into the mass spectrometer using the gas or liquid stream, wherein preferably the stream comprises a continuous stream, continuous infusion, segmented flow and/or flow injection, preferably a continuous stream.

Preferably, the sample transfer system is configured to ionize the sample, preferably prior to or during ejection of the sample. Preferably, the sample transfer system comprises an element located directly above the sample, wherein the element is configured to have a high voltage. Preferably, the voltage is of from 500 to 5000 V.

Preferably, the sample transfer system comprises a system that is configured to introduce a sample or an aliquot thereof to a mass spectrometer comprising a sampling probe having an open end presenting an air interface for receiving the sample, an inlet connected to the sampling probe supplying a gas or liquid stream capable of entraining the sample, an outlet connected to the sampling probe receiving the gas or liquid stream, wherein the outlet is configured to be in fluid communication with a mass spectrometer. Preferably, the gas or liquid stream is comprised by the carrier.

Preferably, an aliquot is ejected and directed from the sample towards an opening of the mass spectrometer, such as e.g. an open port probe, wherein the aliquot is immediately entrained in a gas or liquid stream comprising a a solvent. The aliquot is then transported and processed according to conventional mass spectrometry methods.

An example of a commercial system employing a system comprising a sampling probe having an open end as described above (also termed "open port interface") coupled with an acoustic (i.e. sonic) aliquot ejection system (also termed "acoustic droplet ejection") and a mass spectrometer is the Echo^{®} MS system manufactured by SCIEX, which is a preferred system to execute the method according to the invention.

The inlet and the outlet according to the invention can be positioned relative to each other in various configurations known in the art. For example, the inlet and the outlet can both be a tube, wherein the the inlet surrounds the outlet at their connection to the sampling probe. The gas or liquid stream may be configured in such a way that the rate of the supply via the inlet and the rate of removal via the oulet differ from each other, thereby generating a gas or liquid stream that is in a vortex or meniscus configuration at the open end. To this end, preferably, the rate of supply is greater than the rate of removal, thereby generating a gas or liquid stream that is in a meniscus configuration, preferably a convex meniscus. More preferably, the rate of removal is greater than the rate of supply, thereby generating a gas or liquid stream that is in a vortex configuration, preferably a critical or supercritical vortex configuration.

In order to achieve a flow rate of the gas or liquid stream and subsequent aliquot analysis throughput that is at a rate sufficiently high for industrial applications using a sampling probe having an open end presenting an air interface for receiving the sample according to the invention, it is advantageous to reduce any negative capillary and Venturi effects that are generated by the gas or liquid stream. This can be achieved by a person skilled in the art by for example, in the sample transfer system or the assaying system, varying the respective diameters, lengths and/or materials of the transporting means of the inlet and/or outlet or by adjusting, preferably increasing, the pressure of the gas or liquid streams.

In addition, preferably, the automated egg determining system further comprises a light source emitting light in a wavelength of from 10 to 400 nm to inactivate pathogens residing in or on the egg or the egg determining system.

Preferably, the characteristic is determined based on analyzing the amount of an analyte in the sample or an aliquot thereof wherein the analyte comprises a steroid or its derivatives.

Preferably, the characteristic is the sex of the embryo in the egg. Preferably, the characteristic is the feeding state, health status or developmental status of the embryo in the egg. Other preferred non-limitative implementations of the automated egg determining system are disclosed below.

Preferably, the automated egg determining system comprises a system to run an algorithm to exclude unfertilized eggs or eggs containing dead, un- or underdeveloped embryos.

Preferably, the automated egg determining system comprises a sorting system in communication with the assaying system and is configured to sort the egg based on the characteristics of the egg as determined by the assaying system. This enables a selection of certain eggs according to their determined characteristics.

Preferably, the sorting system comprises a holding area configured to hold and receive the egg for a predetermined period of time.

Preferably, the sorting system comprises an egg marking system, which comprises a means to mark an egg on the outside of the egg in line with the characteristic.

Preferably, the sampling system comprises a candling unit which comprises one or more light sources and one or more detectors. This enables a visual or automatic determination of specific structures of the egg or the embryo inside the egg.

Preferably, at least one detector is positioned relative to the light source such that the detector can detect an image of the egg via light originating from the light source. Light from a light source can traverse the egg and/or reflect in any direction from the egg or the inside of the egg.

More preferably, the light source is positioned between the egg and the detector.

Preferably, the detector is positioned at an angle of from 0 to 45° relative to a light ray originating from the light source. This enables the generation of a stronger signal by the detector.

Preferably, the egg is positioned at a distance of from 0-30 mm from the light source of the candling unit for candling. This short distance reduces the scattering of incoming light to the egg and reflection of light from other surfaces. Therefore, less light is needed to produce a better visualization of the egg and hence determination of specific structures of the egg or the embryo inside the egg. In addition, the reduced amount of light energy emitted towards the egg minimizes adverse effects of the light or the light energy on the egg or the embryo inside the egg.

Preferably, the light source comprises an incandescent or luminescent light source.

More preferably, the light source comprises a halogen, gas-discharge, laser or light-emitting diode light source.

Preferably, the light source comprises a high-intensity discharge, fluorescent, neon, argon, sulfur, metal-halide, plasma, xenon-flash, laser diode, chemical laser, gas laser, ion laser, solid-state laser light source.

Preferably, the light source is configured to emit light in a wavelength of from 300 to 2500 nm.

Preferably, the candling unit comprises a spacer system comprising a spacer object. This enables the positioning of the egg at a set distance.

Preferably, the system is configured to, and operable to position the egg to be in contact with the spacer object prior to, or during candling.

Preferably, the spacer system comprises a light source. This reduces the scattering of incoming light to the egg and reflection of light from other surfaces.

Preferably, the spacer object is of an essentially tubular shape, and located between the egg and the light source and comprises a lumen with two openings, one of which is opposite the egg, the other which is opposite the light source. This enables a channel through which light from the light source can reach the egg and thereby reduces the scattering of incoming light to the egg and reflection of light from other surfaces. Therefore, less light is needed to produce a better visualization of the egg and hence determination of specific structures of the egg or the embryo inside the egg. In addition, the reduced amount of light energy emitted towards the egg minimizes adverse effects of the light or the light energy on the egg or the embryo inside the egg.

Preferably, the spacer object has a spacing thickness in the range of from 0.01 to 20 mm. This short distance reduces the scattering of incoming light to the egg and reflection of light from other surfaces.

More preferably, the spacer object is compressible at a compressive strength in the range of from 0 to 5 MPa, and optionally, exhibits material with an external shore hardness of from 0 to 90 Shore OOO. The low compressive strength and shore hardness of the spacer object are important for minimizing any potential damage to the egg and specifically the egg shell when the egg is brought into contact with the spacer object.

Preferably, the spacer system comprises a spring configured to sustain the spacer in contact with the egg surface when compressed. This is important for minimizing any potential damage to the egg and specifically the egg shell when the egg is brought into contact with the spacer object.

More preferably, the spacer system comprises a bearing allowing rotation around a central axis. This allows (re)positioning of the egg for better access to specific structures of the egg or the embryo inside the egg during sampling of the egg.

Preferably, light originating from the light source present in the spacer system exits the spacer system via one opening. This enables the direction and focus of the light towards the egg.

Preferably, the system is configured to, and operable to position the egg to be in lateral contact with one or more, preferably three or four, movable objects configured to move into or out of contact with the egg to respectively hold the egg or release the egg, preferably prior to, or during candling. This ensures that the egg is directed upwards aligning the central egg axis of the egg with the direction of gravity. When the egg is held into contact during candling a steady image of the egg can be captured by a detector. Release of the contact enables the egg to be (re)positioned.

More preferably, the movable objects comprise an elastic object that stores mechanical energy.

Preferably, the elastic object that stores mechanical energy comprises a spring selected from the group consisting of a flat spring, a leaf spring or a coil spring.

Preferably, the sampling system comprises a means to determine the location of the air cell of the egg.

Preferably, the sampling system comprises a means to determine the location of the allantois of the egg.

Preferably, the sampling system comprises a means to determine the location of a preferred extraction point of the egg. This enables an access location where one or more samples can be taken safely from one or more specific desired structures.

More preferably, the location of a preferred extraction point on the egg shell comprises a point on the shell of the egg on a line parallel to the central egg axis having a distance of from 0.5 to 7 mm to an intersection of the air cell with the allantois, the intersection being within a distance of from 0 to 2 mm from the shell of the egg, the distance to the intersection measured in the direction perpendicular to the central egg axis.. This enables an access location to the air cell or any directly or indirectly adjacent structures.

Preferably, the sampling system comprises one or more openers to open a part of the egg shell of one or more eggs.

Preferably, the sampling system is configured to position the egg and the opener to bring the opener and/or the egg into contact with the opener at the preferred extraction point, or to position the egg and/or the opener in the trajectory of the opener movement towards the preferred extraction point.

More preferably, the sampling system is configured to position the egg and/or the opener, based on the central egg axis and the opener trajectory, at an angle of from 0 to 90°, or preferably 15 to 90°, with respect to the direction of the opener trajectory towards the preferred extraction point. This enables an ideal angle to minimize the amount of energy required to open the egg, thereby minimizing the risk of damaging the egg or any structures inside the egg.

Preferably, the sampling system comprises one or more extractors to extract a sample from one or more eggs.

More preferably, the sampling system comprises one or more extractors to extract a sample from the allantois of one or more eggs.

Preferably, the sampling system is configured to position the egg and extractor relative to each other to bring the egg and extractor into contact at the preferred extraction point, or to position egg and/or extractor in a position in line with the trajectory of the extractor towards the preferred extraction point.

More preferably, the sampling system is configured to position the egg and/or extractor, respectively, based on the central egg axis at an angle of from 0 to 90°, or preferably 0 to 45°, with respect to the extractor trajectory towards the preferred extraction point. This enables an ideal access and access trajectory of the extractor to specific desired structures of the egg for sampling.

Preferably, the extractor is configured to, and operable to traverse the air cell of the egg by a distance in the range of from 0.5 to 9 mm, preferably 3 mm, and to enter the allantois of the egg.

Preferably, the extractor is configured to remove a volume of from 100 nl to 500 µl from the egg.

Preferably, the sampling system comprises a system to clean the extractor before or after extracting one or more samples from the egg. This is to reduce contamination of the eggs by pathogens and to reduce cross-contamination of samples from the eggs.

Preferably, the sample transfer system comprises a liquid handling robot.

Preferably, the sample transfer system is configured to hold and transfer the sample via one or more instruments for holding comprising a multitude of sample containers or via one or more instruments for holding a multitude of sample containers. This enables the hold and transfer of the sample in, for example, a microtiter plate for further processing or analysis at a later stage.

Preferably, the sample transfer system is configured to contact the sample or an aliquot thereof with other materials. This to enable dilution and/or reaction of the sample with other reagents or solid material necessary to determine the amount and/or presence of one or more analytes for determination of one or more characteristics of the sample and by extension the egg or the embryo inside the egg. The reaction with other reagents comprises methods for purification, concentration and/or isolation of materials from the sample or an aliquot thereof, such as for example solid-phase extraction.

Preferably, the sample transfer system is configured to contact the sample or an aliquot thereof with a known amount of reference material. This to enable the determination of the amount and/or absence or presence of one or more analytes for determination of one or more characteristics of the sample and by extension the egg or the embryo inside the egg. Preferably, the sample transfer system is configured to blend the sample or an aliquot thereof with other materials or a known amount of reference material. Preferably, the sample system is configured to blend by applying minimal force in order to prevent the formation of air bubbles.

Preferably, the sample transfer system or assaying system comprises segmented flow or flow injection. This enables an automated and segmented in-line transfer and/or reaction of samples prior to analysis.

The samples and/or the analytes therein are preferably analyzed in a high-throughput method so that many samples can be analyzed in a short period of time. To allow high-throughput measurements, the samples and aliquots thereof, and eventually purified and/or diluted aliquots thereof are generated as part of the automated system. Therefore, after the sampling step, the samples are optionally purified for injection into a mass spectrometer for analysis.

The sampling and analysis may be done in-line, or off-line with a parallel purification and measurement system, thereby allowing a high-throughput mass spectrometry analysis by not sequentially tying to, and hence and slowing down the automated mass spectrometry analysis. The system allows for off-line parallel purification of the samples with no time-consuming separation processes.

The purification may also be performed as part of the sampling process. In this way the system allows all samples to be sufficiently purified for mass spectrometry analysis without a column separation that is performed sequentially and in-line with the mass spectrometer.

To do this the system may provide a chemical and/or physical purification step that is selected based on the type of sample. Furthermore, the purification step can be performed at any stage suitable in-line, or parallel off-line system.

For example, the off-line chemical purification step optionally comprises the use of a buffer or otherwise suitable diluent, or the use of a filtration.

Preferably, the assaying system comprises a system to detect molecules with a concentration of from 10⁻⁷ mol/m³ to 10⁻² mol/m³ in a sample.

Preferably, the assaying system is configured to generate a test result from an assay for detecting an analyte in a sample or an aliquot thereof, optionally contacted with other materials, within a time period of from 0.1 to 6 seconds after receiving the sample or an aliquot thereof from the sample transfer system.

Preferably, the sample transfer system is configured to transfer a sample, or an aliquot thereof, optionally contacted with other materials, of a volume of from 1 to 1000 nL to the assaying system.

Preferably, the assaying system comprises one or more mass spectrometers, gas chromatographs, ion-mobility spectrometers, nuclear magnetic resonance spectrometers, Raman spectrometers, infrared spectrometers or electronic noses.

Preferably, the assaying system comprising a mass spectrometer further comprises electrospray ionization, matrix-assisted laser desorption/ionization or atmospheric-pressure chemical ionization.

Preferably, the electronic nose comprises one or more sensors which comprise one or more of the following types: a protein which binds specific molecules, a metal-oxide-semiconductor, a conducting polymer, a polymer composite, a quartz crystal microbalance or a surface acoustic wave.

Preferably, the sample transfer system comprises a sample aspiration tube and an injection valve, the injection valve being configured to alternatively apply a reduced pressure to a first fluid source and to a second fluid source, in each case via the sample aspiration tube, the first fluid source for filling a sample loop with samples, and the second fluid source for flushing the aspiration tube.

Preferably, the sample transfer system comprises a system that is configured to transfer the sample or an aliquot thereof, optionally contacted with other materials, according to the following steps:
a. an aliquot from the sample is ejected by applying energy to an amount of the sample;
b. the ejected aliquot is entrained in a gas or liquid stream; and
c. the entrained aliquot is transported into the mass spectrometer using the gas or liquid stream.

Preferably, the energy is sound or light energy. Preferably, the energy is light energy. More preferably, the energy is sound energy. Preferably, the sample transfer system comprises a system that is configured to introduce a sample or an aliquot thereof to a mass spectrometer comprising a sampling probe having an open end presenting an air interface for receiving the sample, an inlet connected to the sampling probe supplying a gas or liquid stream capable of entraining the sample, an outlet connected to the sampling probe receiving the gas or liquid stream, wherein the outlet is configured to be in fluid communication with a mass spectrometer.

Herein, an aliquot may then be ejected and directed from the sample towards an opening of the mass spectrometer, e.g. an open port probe) wherein the aliquot is immediately entrained in a gas or liquid stream comprising a solvent. The aliquot is then transported and processed according to conventional mass spectrometry methods.

Preferably, the assaying system is configured to generate a test result from an assay for detecting an analyte in the sample or an aliquot thereof, optionally contacted with other materials, comprising the following steps:
a. the analyte is ionized; and
b. the amount of analyte is detected by a mass spectrometer, wherein the amount of analyte is related to the amount of analyte in the sample or an aliquot thereof or a known amount of reference material in the sample or an aliquot thereof.

Preferably, the sampling system is in a fluid connection with the sample transfer system. This enables direct in-line transfer of samples from the sampling system to the sample transfer system, thereby reducing the time required for transfer and any influence of external factors on the sample.

Preferably, the sample transfer system is in a fluid connection with the assaying system. This enables direct in-line transfer of samples from the sample transfer system to the assaying system, thereby reducing the time required for transfer and any influence of external factors on the sample.

Preferably, the sample transfer system is in a fluid connection with the sampling system and the assaying system. This enables direct in-line transfer of samples from the sampling system to the assaying system, thereby reducing the time required for transfer and any influence of external factors on the sample.

An example relates to a method for determining a characteristic of one or more eggs, comprising the steps of:
a. automatically extracting a sample from one or more eggs;
b. transferring the sample via in-line transfer; and
c. automatically analyzing the sample or an aliquot thereof in a mass spectrometer for the absence or presence and the amount of one or more molecules, to determine one or more characteristics of the egg.

Preferably, the one or more molecules comprise a biomarker, a veterinary drug or a pesticide. More preferably, the one or more molecules comprise a biomarker.

Preferably, step a. comprises determining the location of the air cell, the allantois and/or a blood vessel of the egg.

Preferably, the sample comprises an allantoic fluid sample or a blood sample.

Preferably, extracting the sample comprises expelling the sample from the interior of the egg to the exterior of the egg as a result of controlling pressure in the interior of the egg and collecting the sample or an aliquot thereof at the exterior surface or interior-exterior intersection of the egg.

Preferably, controlling pressure comprises engaging a contact area of the egg shell, preferably at the air cell.

Preferably, step a. further comprises determining the location of a preferred sample extraction point in the egg or at the exterior surface of the egg and collecting the sample or an aliquot thereof at the preferred sample extraction point.

There is also provided a method for determining eggs preferably involving the steps of:
a. candling one or more eggs;
b. automatically extracting one or more samples from one or more eggs;
c. transferring the sample to be analyzed;
d. automatically analyzing the sample or an aliquot of a sample thereof and determining one or more characteristics of the egg.

Preferably, the method comprises an additional step of positioning the egg in front of a light source and in contact with a spacing system preferably prior to, or during candling.

Preferably, the method comprises an additional step of determining the location of the air cell of the egg.

Preferably, the method comprises an additional step of determining the location of the allantois of the egg.

Preferably, the method comprises an additional step of determining the location of a preferred extraction point of the egg.

There is additionally provided the use of a system for automated determination of a characteristic in a multitude of eggs according to any of the systems described above.

The terms "automated" or "automatical" refer to the use of a system that can operate autonomically, without user interaction being required, and permitting to sample and measure multitudes of eggs, such as e.g. 1000 to 10.000 eggs per hour.

The present system preferably hence comprises at least one autosampler coupled with the apparatus of the invention to transport samples between the sampling system, an optional purification step, and a mass spectrometer for injection and analysis. Suitable autosamplers are well known, and usually function at rates of about 1 second/sample to about 15 seconds/sample.

In addition, robotic sampler handlers are optionally used to sample, and to add reagents in the optional inline or off-line parallel purification system.

For the generation of common arrangements involving fluid transfer to or from microtiter plates, a fluid handling station may advantageously be used. Such robotic handlers may utilize automatic pipettors, e.g., in conjunction with the robotics for plate movement.

Particularly relevant molecules are those that can be used as a biomarker for determining certain characteristic, such as gender, developmental status, viability, health or the like.

Such molecules include those disclosed, for example, in WO9814781A1, i.e. wherein it is disclosed that the presence or absence of an elevated level of a sex-related hormone in the allantoic fluid of the bird egg may permit to determine the gender of the bird within the egg from the presence of an elevated level of a sex-related hormone therein.

The sex-related hormone preferably is a natural, endogenous steroid, more preferably an estrogen ester and conjugate, yet more preferably a salt of estrone, such as estrone sulfate, i.e. 1,3,5(10)-Estratrien-17-one-3-sulfate. Other suitable molecules may comprise gender-linked proteins, nucleotides, and the like. Another preferred biomarker according to the invention is 3-[(2-aminoethyl)sulfanyl]butanoic acid, also referred to herein as Biomarker 1.

### Analysis method and threshold determination:

The present disclosure further presents a method for analyzing the sample or an aliquot thereof in an automated manner in a mass spectrometer for the absence or presence and the amount of one or more molecules, to determine one or more characteristics of the egg.

Determining the one or more characteristics of the egg by analysing the sample or an aliquot thereof for the absence or presence and the amount of one or more molecules results in the identification of different populations of eggs having different characteristics. However, it may be difficult to determine the characteristic of certain eggs based on the amount of one or more molecules when, for example, a certain amount or range of the one or more molecules does not result in a certain identifcation of the characteristic; this can occur when there is an overlap of a range of concentrations of a molecule, where the molecule cannot identify a characteristic with full certainty. It is common in the art to use a cut-off or threshold value above which one characteristic can reasonably be determined and below which the absence of the one characteristic or a different characteristic can reasonably be determined. In the field of the biological sciences however, such a cut-off value usually results in the generation of false positive and false negative determinations or errors due to the normal (or Gaussian) distribution of many biological molecules in organisms. The false positive rate is the proportion of all negatives that still yield positive test outcomes. The specificity of a determination is equal to 1 minus the false positive rate. The false negative rate is the proportion of positives which yield negative determination outcomes with the determination.

It was found that for the determination of one or more characteristics according to the invention using a traditional theoretical best threshold of peak area ratio or a fixed (a *posteriori-*determined) threshold value yields a substantial error rate. This results in a significant portion of the to be determined eggs to obtain an uncertain determination and therefore these eggs need to be discarded, which leads to an uneconomical method unsuitable for an industrial setting. Specifically, the traditional fixed threshold approach is unsuitable, because of for example interbatch variation, degradation of the reference material or internal standard or evaporation of the solvent(s).

The inventors have found that it is possible to reduce the error rate of the determination according to the invention by determining an improved threshold value, termed the dynamic threshold value as follows.

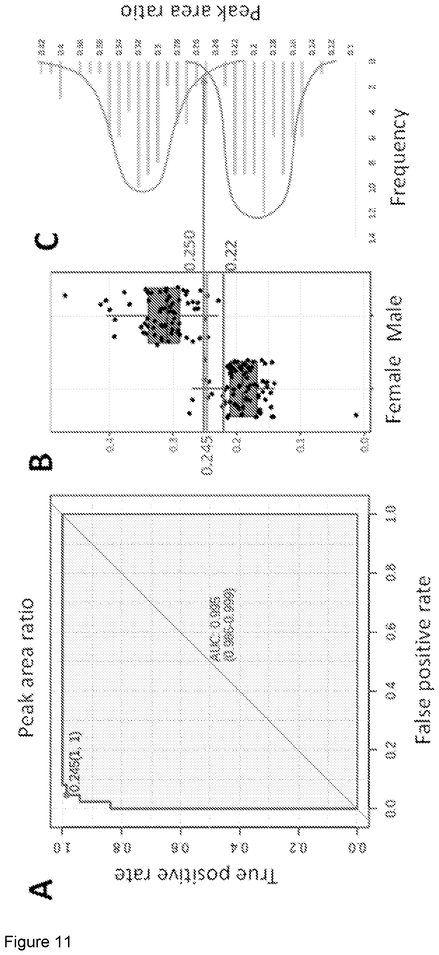

Determining the one or more characteristics of the egg by analysing the sample or an aliquot thereof for the absence or presence and the amount of one or more molecules, comprises using the median of the ratio of the one or more molecules to the reference material. This is particularly useful when the one or more characteristics of the egg is the sex of the embryo, because the expected ratio of male to female is 1 : 1. The median should thus in theory correctly predict a threshold value that separates the male from the female characteristic. This embodiment is exemplified in figure 11B.

Determining the one or more characteristics of the egg by analysing the sample or an aliquot thereof for the absence or presence and the amount of one or more molecules, comprises comparing the amount of the one or more molecules with the amount of the one or more molecules obtained in other analyses according to the method of the invention. Preferably, the other analyses are previous, earlier or historical analyses. Preferably, comparing the amount of the one or more molecules or the amount of the one or more molecules obtained in other analyses comprises comparing the amount of the one or more molecules or the amount of the one or more molecules obtained in other analyses to the reference material, preferably by determining a ratio between the amount of the one or more molecules to the reference material. Preferably, the reference material is the reference material added to or used in the same sample as where the one or molecules to be determined are present. Preferably, comparing the amount of the one or more molecules with the amount of the one or more molecules obtained in other analyses, comprises modelling one or more distribution curves based on the amount of the one or more molecules obtained in other analyses. Preferably, modelling the one or more distribution curves comprises associating one or more characteristics with the one or more distribution curves. Preferably, modelling the one or more distribution curves further comprises determining the intercept of the curves, preferably wherein the intercept is used as the threshold value for determining the one or more characteristics. This embodiment is exemplified in figure 11C.

Preferably, the one or more molecules comprise Biomarker 1. Preferably, the reference material is Biomarker 1-d4 (3-[(2-amino(2D₄)ethyl)sulfanyl] butanoic acid).

The dynamic threshold value as described above can be adjusted by a person skilled in the art based on different amounts of the one or more molecules obtained in different subpopulations of eggs, such as eggs of a different age or size, the feed composition of the parent, the geographical location, temperature or other external factors. A larger amount of other analyses, preferably specific to a subpopulation of eggs, generates a threshold value of greater accuracy.

According to the methods and systems of the invention it is possible to identify a characteristic of an egg comprising obtaining a sample, processing the sample, and analyzing the sample by mass spectrometry in a manner with a sufficient speed and accuracy that is necessary for an industrial setting. As is disclosed in the Examples, the inventors were able to predict the gender of chicken embryos with an accuracy over 95% and an analysis speed above 1800 samples/hour. Sample preparation can be done in parallel to the mass spectrometry analysis, therefore the method or system according to the invention can process more than 2000 samples/hour. Thus, the methods and systems according to the invention show a significant improvement over (mass spectrometry methods and systems of) the prior art.

### Detailed Description of the Figures

The presently claimed invention is as defined in the claims. Any of the following Figures that do not fall within the scope of the claims do not form part of the presently claimed invention and are provided for comparative purposes only. The examples will now be discussed with reference to the figures, which show preferred exemplary embodiments of the subject invention and disclosure.

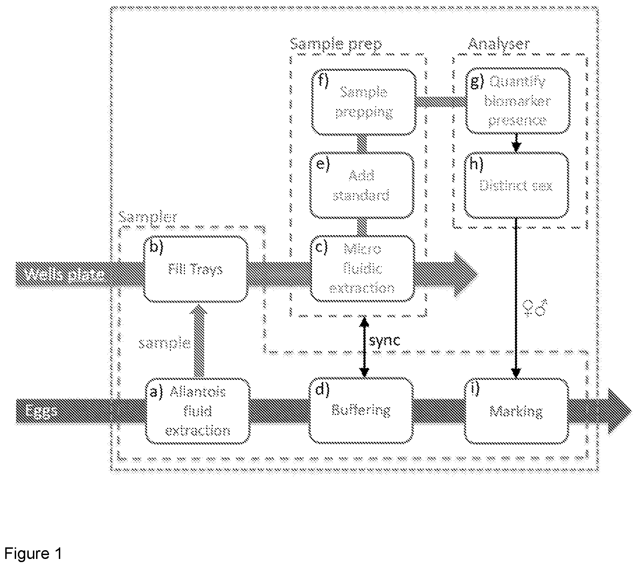

Figure 1 shows a flow chart for a system for taking samples from eggs, transferring the samples to an assaying system and determining one or more characteristics of the eggs. A sample from the allantois (e.g. allantoic fluid) is taken from an egg at step a). The sample is transferred to a microtiter or wells plate in step b), which is used to collect a number of samples from one or more eggs. The samples are optionally contacted with a reference standard material or other materials and optionally blended in steps e) and f), while the egg is held in place in a buffering positioning in step d). An analyser (i.e. a mass spectrometer) runs an assay to determine the level of one or more molecules that may act as a suitable biomarker for determining an egg characteristic in step g) and reports the outcome of the assay for one or more characteristics (e.g. sex) in step h). The egg can then be marked externally for one or more characteristics in step i) for subsequent sorting.

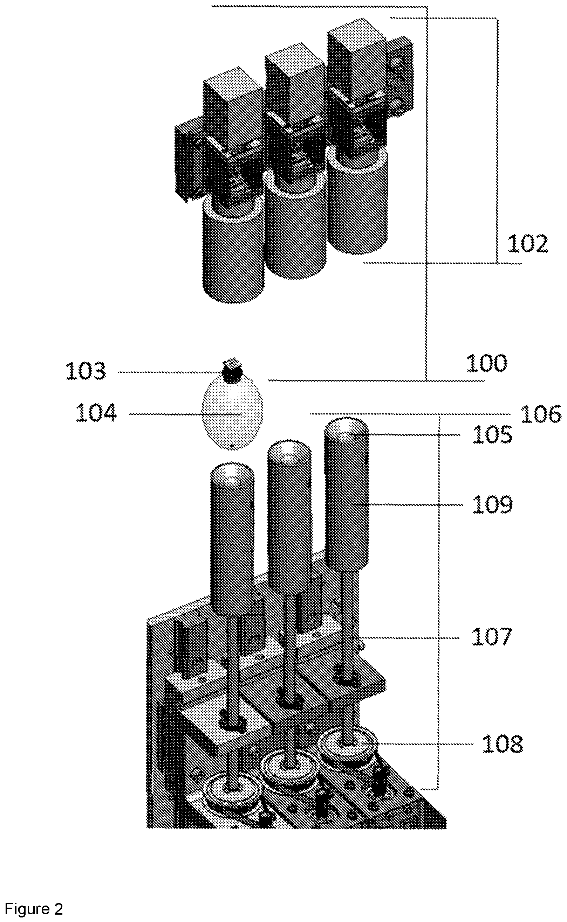

Figure 2 shows a model of a device capable of holding one or more eggs, which is also capable of rotating the egg and/or positioning the egg relative to for example a candling unit. An egg (104) is held in place by a vacuum or a mechanical means (105) of an egg manipulator (106). An extender (107) is configured to position the egg (104) towards a spacer object (103) and in front of a candling unit (100), comprising one or more detectors (102), one or more lighting sources (not shown) and in this model a spacer system (103). In this model the lighting source is located inside the spacer system (103). An egg rotator (108) is configured to rotate the egg manipulator (106) and by extension also the egg (104) and optionally a part of or the whole spacer system (103) when the egg (104) is positioned securely against the spacer system (103) and the spacer system (103) comprises a bearing.

Figure 3 shows a model of an apparatus containing several extractors, capable of moving the extractors from and to an instrument for holding comprising a multitude of sample containers. The apparatus contains one or more extractors (200), in an array, each comprising a hollow elongated object (202). The array comprising the one or more extractors are positioned on a sled which can be moved along a rail (603) in horizontal direction from a position above one or more eggs, towards and from an instrument for holding comprising a multitude of sample containers, such as e.g. a microtiter plate (201). The array (604) can also be moved in vertical direction, driven by a conveyor means (603), This allows for one or more samples to be acquired, and deposited in a container in a fully automated manner.

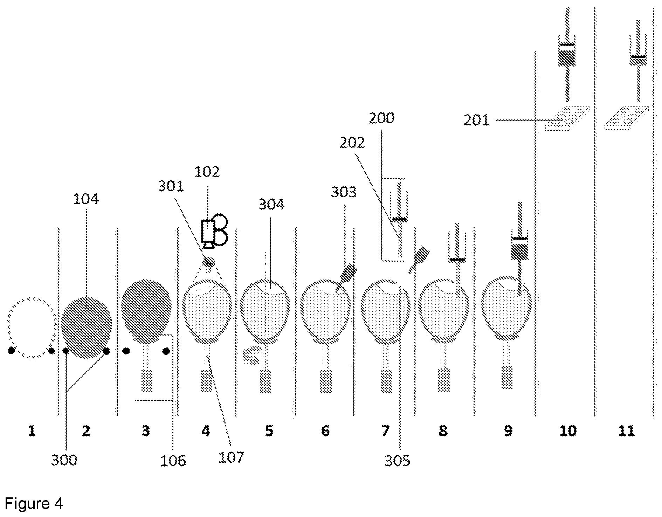

Figure 4 shows a schematic overview of different steps of a system for taking one or more samples from one or more eggs. In step 1 an empty egg holder or tray (300) is present. In step 2 an egg (104) is placed blunt side up on the egg holder or tray (300). In step 3 an egg manipulator (106) is configured to hold the egg securely and is configured to lift the egg (104) from the egg holder or tray (300) via an extender (107). In step 4 the egg (104) is positioned by the egg manipulator (106) in front of a candling unit comprising a light source (301) and a detector (302). (Not shown) The top of the egg (104) is positioned by the extender (107) of the egg manipulator (106) against a spacer system and three or four lateral sides of the egg (104) each are positioned against respectively three or four flat springs. Following this the egg is candled. In step 5 a system which is configured to run an algorithm for determining the location of the air cell (304) of the egg (104) and a preferred extraction point (305) directs the egg manipulator (106) to rotate the egg (104) based on the information gathered by the detector (302). (Not shown) Prior to rotating the egg (104) the three or four lateral springs are moved out of contact with the egg (104). In step 6 the egg (104) and/or an opener (303) are positioned relative to each other in order for the opener (303) and subsequently the extractor (200) to contact the preferred extraction point (305). The opener (303) opens the egg (104). In step 7 the opener (303) is retracted from the egg (104) and the extractor (200) comprising a hollow elongated object (202) is positioned above the preferred extraction point (305). In step 8 the hollow elongated object (202) of the extractor (200) passes the preferred extraction point (305), enters the egg (104) and traverses the air cell (304). The distal end of the hollow elongated object (202) enters the allantois or a different structure of the egg (104). In step 9 the extractor (200) removes a sample from the egg (104). In step 10 the extractor is removed from the egg (104) towards an instrument for holding comprising a multitude of sample containers (e.g. a microtiter plate) (201). In step 11 the extractor (200) ejects the sample in the microtiter plate (201).

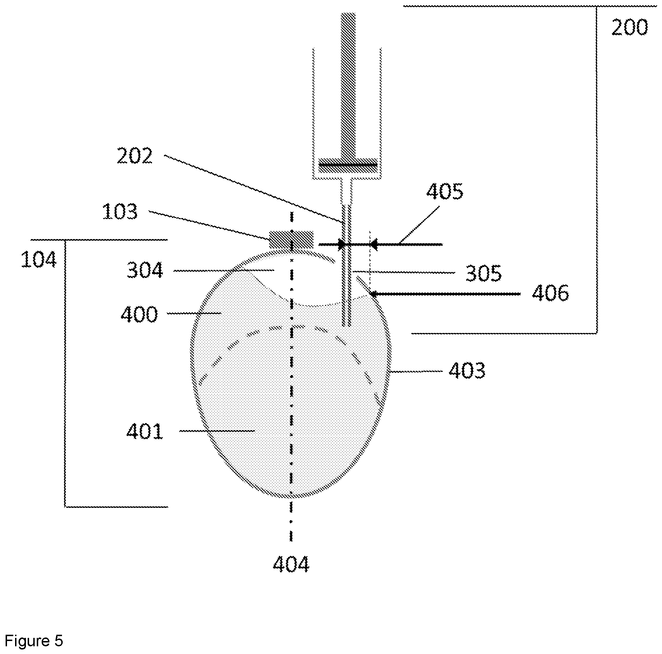

Figure 5 shows a detailed schematic cross section of an extractor positioned in a (chicken) egg at the moment before taking a sample. The hollow elongated object (202) of an extractor (200) has entered an egg (104) through a preferred extraction point (305) and traversed the air cell (304) in order to remove a sample from the allantois (400) of the egg (104) without entering any underlying anatomical structures (401). The blunt end of the egg (104) is positioned against a spacer system (103). The location of the preferred extraction point (305) comprises a point on the shell (403) of the egg (104) on a line parallel to the central egg axis (404) having a distance of from 0.5-7 mm to an intersection (406) of the air cell (304) with the allantois (400), the intersection being within a distance of from 0-2 mm from the shell (403) of the egg (104), the distance to the intersection measured in the direction perpendicular to the central egg axis (404). This distance is the edge distance (405).

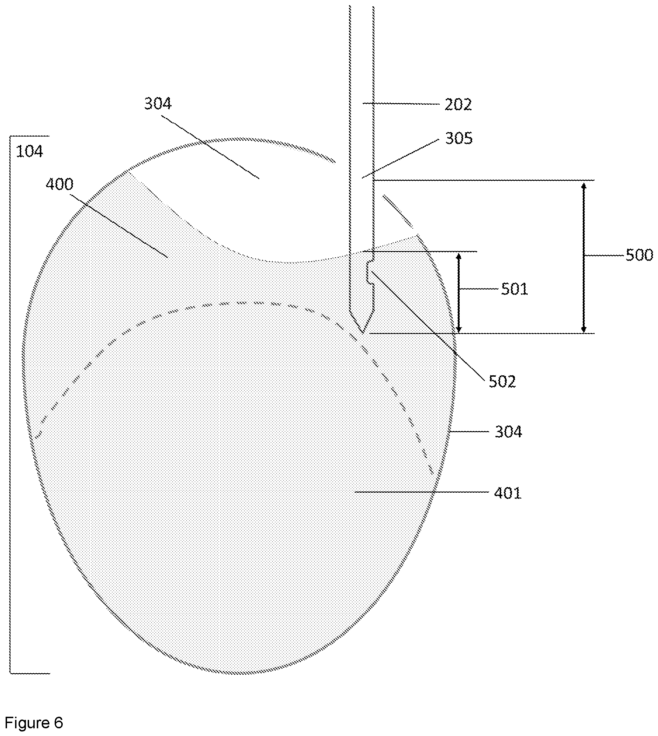

Figure 6 shows a schematic cross section of a hollow elongated object of an extractor positioned in an egg. The hollow elongated object (202) of an extractor (200) has entered an egg (104) through a preferred extraction point (305) and traversed the air cell (304) in order to remove a sample from the allantois (400) of the egg (104) without entering any underlying anatomical structures (401). The distance of which the hollow elongated object (202) has traversed through the egg (104) from the preferred extraction point (305) is termed the needle depth (500). The distance of which the hollow elongated object (202) has traversed the allantois (400) is termed the penetration depth (501). The hollow elongated object (202) comprises a lateral opening (502) configured to remove a sample from the allantois (400).

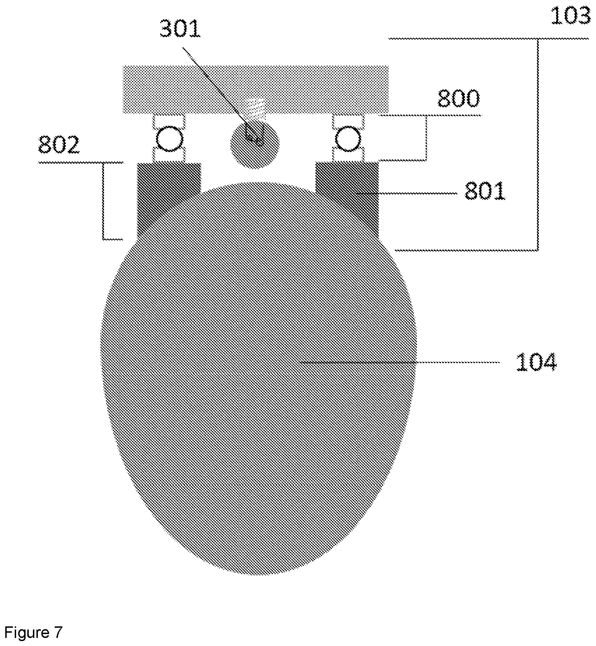

Figure 7 shows a schematic overview of the egg and a spacer system during candling. The blunt side of an egg (104) is positioned against a spacer object (801) of the spacer system (103). The spacer system (103) comprises a light source (301) and a bearing (800). The spacer object has a thickness (802) and comprises an opening towards the egg (104) and an opening towards the light source (301). Light from the light source (301) exits the spacer system (103) only via the egg (104), thereby reducing reflection of the light from unwanted structures and enabling detection of structures within the egg by a detector (not shown).

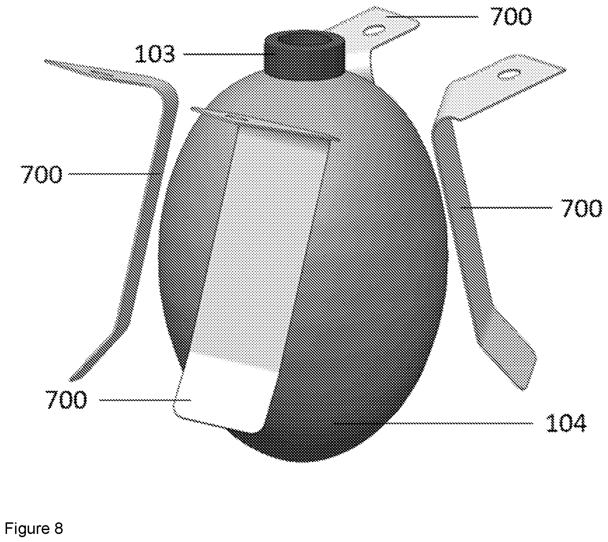

Figure 8 shows a schematic of the egg in contact with a spacer object and out of contact with flat springs prior to being rotated. The blunt side of an egg (104) is positioned against a spacer system (103). Immediately prior to rotating the egg (104) and after candling each of the lateral springs (700) are moved out of contact with the egg (104). (Not shown) Prior to and during candling the egg is positioned to be in contact with all lateral springs (700) enabling the positioning of the central egg axis parallel to the direction of gravity.

The present system permits an automated analysis of a multitude of eggs. Based on a prototype embodiment, a speed of processing is considered feasible in the range of from several hundreds to several thousands of eggs per hour, with an unprecedented selection exactness.

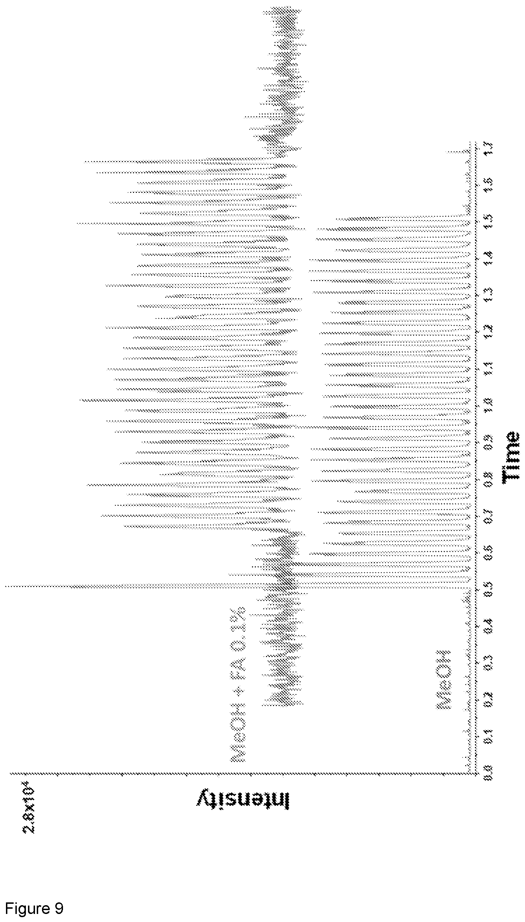

Figure 9 shows a comparison of signal intensities during mass spectrometry analysis of different solvent compositions of the gas or liquid stream according to the invention wherein the one or more molecules comprises Biomarker 1. An aliquot comprising Biomarker 1 (164.1 → 147.0) acoustically ejected from a pooled untreated allantoic fluid sample was entrained in a stream via an open port and transferred according to the invention to a mass spectrometer. The top line shows the signal intensity over time of Biomarker 1 as recorded by the mass spectrometer after the aliquot was entrained and transferred in a stream comprising methanol and 0.1% formic acid, while the bottom line shows the signal intensity in a stream comprising solely methanol as a solvent and no formic acid. The signal to noise ratio of the top line is about 5 and therefore below the limit of quantification, while the signal to noise ratio of the bottom line is about 50.

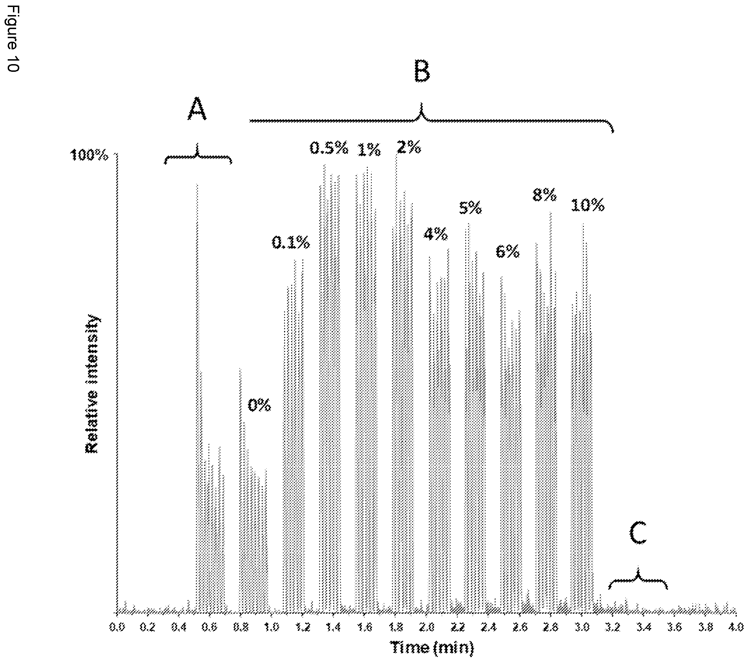

Figure 10 shows the influence of sample dilution and the amount of formic acid added to the sample on the signal intensity during mass spectrometry analysis. Aliquots obtained according to the invention by acoustic ejection from allantoic fluid samples were analyzed in a mass spectrometer after entrainment in the stream via an open port. The relative intensity over time of Biomarker 1 (164.1 → 147.0) comprising aliquots obtained from undiluted allantoic fluid samples is indicated by A, of Biomarker 1 comprising aliquots obtained from allantoic fluid samples that were diluted 1 : 1 with a solution of water containing 0, 0.1, 0.5, 1, 2, 4, 5, 6, 8 or 10% formic acid (w/w) are indicated by B, of aliquots obtained from a sample containing 1% formic acid in water are indicated by C. The difference in signal to noise ratios of the formic acid containing aliquots compared to figure 9 can be explained by the fact that after ejection of the aliquot from the formic acid containing sample and subsequent entrainment into the stream, the formic acid is diluted to such an extent that it has no or at least a greatly reduced influence on the signal to noise ratio.

Figure 11 shows three different methods for determining the sex of the embryo of the egg using the method for determining according to the invention, wherein an aliquot was acoustically ejected and entrained via an open port, wherein the one or more molecules is Biomarker 1 and wherein the reference material is Biomarker 1-d4. (A) shows a receiver operating characteristic (ROC) plot describing the male sex prediction performance of samples obtained according to the method of the invention, wherein the aliquot is acoustically ejected and entrained in a stream via an open port, using a traditional ideal a *posteriori* threshold value. The theoretical best threshold of peak area ratio for this particular batch was found to be 0.245, with a sensitivity of 98.5% (IC95%=94.1-100%) and a specificity of 94.2% (88.4-98.8%). (B) shows a classification of the peak area ratio according to the true sex as determined by PCR analysis of the samples and by evaluating the median ratio Biomarker 1/Biomarker 1-d4. A sensitivity of 100% (IC95%=100-100%) and a specificity of 89.5% (IC95%=80.2-92.4%) were found, resulting in an overall gender prediction accuracy of 94.6%. Consequently, although all the male embryos were correctly called, 10.5% of the female chicks were misassigned. (C) shows the population frequency of the peak area. The bottom and the top curve represent a simulation of respectively the female and male distribution among the sample cohort. The intercept of both curves can be used as the dynamic threshold value. Here, the dynamic threshold value was found to be 0.250. Using this dynamic threshold, a sensitivity of 94.1% (IC95%=86.0-98.5%), a specificity of 96.5% (IC95%=91.9-100%) and a global prediction accuracy of 95.3% was found.

The following is a non-limitative example that illustrates the method and system according to the invention.

### Example 1

### Methods, system and materials

Samples: Allantois samples were manually collected by puncture with an insulin syringe, at day-9 from brown chickens (VB1636 Brown Nick). Approximately 1 mL of allantoic fluid was collected from each egg. After collection, samples were frozen in liquid nitrogen. After their transport from the hatchery to the laboratory, the allantoic samples have been stored at -80 °C prior to analysis. In order to determine the true gender of the embryos, toes were collected and were subjected to PCR DNA analysis. A pooled sample for method optimization was prepared by merging an equal volume of allantoic fluid coming from 150 eggs. To mimic a routine industrial set-up situation, an automatic egg sampler was employed that punched the egg shells and collected approximatively 50 µL of allantoic fluid from each egg in a 96 wellplate.

### High throughput sample preparation

Sample preparation was performed using an Agilent Bravo liquid handling platform. In the same tips, 15 µL of a solution containing 600 ng/mL of internal standard (IS) in 2% formic acid (FA) was aspirated, directly followed by 15 µL of sample. The tip content was then dispensed into an acoustic droplet ejector (ADE) compatible 384 well plate (ABA2-11000A with TC treatment purchased from Aurora Microplates, Whitefish, MT, USA, and mixed by 10 iterations of gentle aspiration and dispense in order to avoid the formation of air bubbles. The final concentration of the sample was 300 ng/mL of IS and 1% FA. Then 10 µL was aspirated, to ensure wetting of the wall of the wells over an area large enough to promote the formation of a horizontal meniscus.

### ADE - open port probe (OPP) - mass spectrometer (MS)

An ADE-OPP-MS system was used based on an acoustic liquid handling, comprising a modified EDB Biosystem Gen4+ where the rotary function of the receiver plate had been disabled and an open port probe interface had been mounted at 2 mm from the source plate. The OPP was centered over the acoustic transducer central axis within ±100 µm using the ATS-100 software and 5 nL droplets were generated using the water calibration and a pause of 1.2 s was observed between injection of each sample.

An LC 1260 series from Agilent (Waldbronn, Germany) was used to deliver make-up solvent at 200 µL/min. A fused silica capillary of 160 cm and 50 µm i.d. from BGB Analytik (Harderwijk, The Netherlands), was used to generate 200 bar back- pressure.

The ADE-MS system was hyphenated to a 6500+ QTrap (AB Sciex, Concord, ON, Canada), equipped with an Optiflow source. MS experiments were performed in the positive ionization mode using the selected reaction monitoring (SRM) acquisition mode. The precursor and product ions that were monitored for each compound, as well as the respective collision energies, are reported in Table 1.

The solvent transfer tube was a PEEKsil, having dimension of 800 µm OD × 250 µm ID × 60 cm length, and was vertically aligned in the center of the OPP. It connects with a 250 µm ID union to a modified stainless-steel electrode/probe assembly. The ion source gas 1 and 2 pressures were fixed at 90 and 85 psi, respectively, with a temperature of 300 °C for both.

The ion spray voltage, declustering potential and collision cell exit potential were adjusted to 4000 V, 80 V and 10 V, respectively. The curtain and collision gas were set at 25 psi and "medium", respectively. Data acquisition and instrument control were monitored using AB Sciex Analyst version 1.6.2 (AB Sciex, Concord ON, Canada).

**Table 1: MRM transition list**

| | Precursor m/z | Fragment m/z | Fragmentation voltage | Dwell time (ms) |
|---|---|---|---|---|
| Biomarker 1 | 164.1 | 147.0 | 14 | 40 |
| Biomarker 1-d4 | 168.1 | 151.0 | 14 | 40 |

### ADE-MS data processing

ADE-MS technology allows acquisition at a very high frequency. Therefore, it is impossible to directly obtain a data file for each sample and instead, the data from all the samples in a plate are present in the same raw data file. For the processing of data acquired with this prototype, it is necessary to split the file in order to generate individual results for each sample. This splitting step was performed by detecting the peak corresponding to the injection of the first sample using a threshold based on the intensity of the most intense peak present. Then, the ADE report containing the time interval between two consecutive ejection events (i.e. two consecutive samples) was used to assign each data point of the chromatogram to a specific sample and finally to split the raw data to individual files.

### Results

The embryo gender assay was set-up to measure at least 1800 samples per hour at an accuracy to reach a minimal gender prediction accuracy of 95%. Female embryos presented an about 30% lower concentration of Biomarker 1 than male embryos, with a border concentration estimated at 100 ng/mL sample selected for the present method when applied on day 9 eggs.

However, due to biological and analytical variations, the highest female and the lowest male concentrations were separated by a concentration difference of less than 10%.

Accordingly, all steps of the process require careful calibration of the sample collection, in particular when employing a high throughput sample and data acquisition and processing method, permitting to accurately interpret the data and predict the gender of the embryos.

### ADE-MS optimization

Acoustic droplet dispensing requires that the depth of the liquid to be dispensed is measured for the focal point of the sound waves to be mechanically set to irradiate the correct area of the liquid surface to achieve the targeted volume. To do this, the acoustic transducer will measure the liquid level in the well by sending an acoustic wave to the sample plate through a coupling liquid (i.e. water) and measuring the interval of time between the reflected signal of the well-sample interface and the meniscus of the sample. After its ejection, the droplets fly with a vertical motion and are captured by the OPP into a stream flow of solvent and carried to the MS through the transfer capillary. Droplets can be ejected at a frequency of 800 Hz.

In order to increase the sensitivity of the method, different sample compositions were tested. As shown in Figure 10, raw samples and samples diluted 2-times with pure water give approximatively the same signal for the endogenous level of Biomarker 1.

For method robustness purposes, a 2-times dilution of the allantoic fluid with water containing 2% of formic acid, for a final FA concentration of 1% was employed.

Since Biomarker 1 appears to be an endogenous compound and no blank matrix is available, the limit of detection (LOD) and quantification (LOQ) have been estimated by injection of increasing concentrations of Biomarker 1-d4 in a pooled allantoic sample, assuming Biomarker 1 and Biomarker 1-d4 have the same MS response. Therefore, the LOD (S/N>3) was determined as 5 ng/mL while the LOQ (S/N>10) was between 10 and 20 ng/mL which is an acceptable sensitivity considering the threshold concentration between male and female embryos is in the range of 100 ng/mL.

### Sample preparation

In order to improve the robustness of the overall method to meet requirements of an industrial process, Biomarker 1-d4 as a deuterated internal standard (IS). With this approach, it is possible to compensate for intra batch variation of the matrix effect, as well as injection variability and long-term source fouling.

### Threshold determination and prediction accuracy

In order to develop an economically viable gender screening test, a minimum of viable females have to be discarded. Although the prediction capacity of the biomarker is very good, the determination of a cut-off value remains economically difficult since in order to separate the distributions in both genders, the precision in measuring the relative concentration difference of Biomarker 1 between male and female is in a 10% range.

In order to evaluate different threshold approaches, 157 samples, coming from two different batches of day-9 eggs, have been analyzed and processed using the workflow shown herein.

As shown in Figure 11 A, the theoretical best threshold of peak area ratio for this particular batch was determined to be 0.245, with a sensitivity of 98.5% (IC95%=94.1-100%) and a specificity of 94.2% (88.4-98.8%). However, a retrospective threshold determination is not compatible with a routine application and an alternative threshold determination is therefore beneficial. After manual inspection of the data, 0.251 was determined as optimal cut-off value giving the higher prediction rate, i.e. 95.9%. In addition, this value was the best for both batches individually considered, showing this approach could be used whatever the egg flock. However, the fixed threshold approach may still comprise interbatch variation, degradation of the IS or slow evaporation of IS solvent. In order to overcome these issues, a dynamic approach was applied, using statistical parameters specific to each sample plate. First the median ratio Biomarker 1 / IS was evaluated (Figure 11 B).

A second approach was evaluated based on population distributions (Figure 11 C). With a sufficient sample cohort, it becomes possible to distinguish two Gaussian curves in the distributions of the population. As shown in Figure 11 C, the intercept of both curves can be used as the cut-off value. In the present case, although the sample cohort was limited, the dynamic cut-off value was found to 0.250, which is close to the ideal threshold.

To summarise, the above method permits to determine one or more characteristics, in particular the gender of chick embryos with an unprecedented combination of accuracy and throughput.

## Claims

1. A method for determining a characteristic of one or more eggs (104) in an automated manner, comprising the steps of:
a. extracting a sample from one or more eggs in an automated manner;
i. holding and transferring the sample through one or more instruments to a unit for holding one or more samples;
ii. ejecting an aliquot from the sample by applying sound energy via an acoustic transducer or light energy to at least an amount of the sample;
iii. entraining the ejected aliquot in a gas or liquid stream; and
iv. transporting the ejected aliquot to a mass spectrometer using the gas or liquid stream;
and
b. analyzing the aliquot of the sample in an automated manner in the mass spectrometer for the absence or presence and the amount of one or more molecules, to determine one or more characteristics of the egg.

2. The method according to claim 1, further comprising, to step a.,
providing a system that is configured to introduce a sample or an aliquot thereof to a mass spectrometer, the system comprising
i. a sampling probe having an open end presenting an air interface for receiving the sample,
ii. an inlet connected to the sampling probe supplying a gas or liquid stream capable of entraining the sample, and
iii. an outlet connected to the sampling probe receiving the gas or liquid stream, wherein the outlet is configured to be in fluid communication with a mass spectrometer.

3. The method according to claim 1 or claim 2, wherein the aliquot has an average volume of at least 2 nl or of 2 to 10 nl.

4. The method according to any of claims 1 to 3, wherein ejecting the aliquot comprises the sample having a substantially horizontal or concave liquid-air interface surface area.

5. The method according to any of claims 1 to 4, wherein the sample comprises an allantoic fluid sample or a blood sample.

6. The method according to any of claims 1 to 5, wherein the gas or liquid stream comprises a solvent, wherein the solvent comprises methanol and/or wherein the one or more molecules comprises 3-[(2-aminoethyl)sulfanyl]butanoic acid.

7. The method according to any one of claims 1 to 6, wherein step a. further comprises determining the location of a preferred sample extraction point on the egg shell and collecting the sample at the preferred sample extraction point, wherein the location of the preferred sample extraction point on the egg shell comprises a point on the shell of the egg on a line parallel to the central egg axis having a distance of 0.5 to 7 mm to an intersection of the air cell with the allantois, the intersection being within a distance of 0 to 2 mm from the shell of the egg, the distance to the intersection measured in the direction perpendicular to the central egg axis.

8. The method according to any of claims 1 to 7, wherein determining the one or more characteristics of the egg comprises comparing the amount of the one or more molecules with the amount of the one or more molecules obtained in other analyses according to the method of the invention.

9. An automated egg characteristic determination system, comprising:
a. a conveyor system configured to transport one or more eggs (104) to a sampling system;
b. the sampling system being configured to extract a sample from one or more eggs, wherein the sampling system or a part thereof is movable towards a sample transfer system, or vice versa, to transfer the sample between the sampling system and the sample transfer system;
c. the sample transfer system being configured to receive the sample for transfer to an assaying system, wherein the sample transfer system comprises a system that is configured to transfer the sample, wherein the system that is configured to transfer the sample is configured to:
i. eject an aliquot from the sample by applying light energy to an amount of the sample;
ii. entrain the ejected aliquot in a gas or liquid stream; and
iii. transport the entrained aliquot into a mass spectrometer using the gas or liquid stream; and
d. the assaying system comprising the mass spectrometer and being configured to receive the entrained aliquot of the sample from the sample transfer system and to determine one or more characteristics of one or more eggs.

10. An automated egg characteristic determination system, comprising:
a. a conveyor system configured to transport one or more eggs (104) to a sampling system;
b. the sampling system being configured to extract a sample from one or more eggs, wherein the sampling system or a part thereof is movable towards a sample transfer system, or vice versa, to transfer the sample between the sampling system and the sample transfer system;
c. the sample transfer system being configured to receive the sample for transfer to an assaying system, wherein the sample transfer system comprises an acoustic transducer and a system that is configured to transfer the sample, wherein the system that is configured to transfer the sample is configured to:
i. eject an aliquot from the sample by applying sound energy via the acoustic transducer to an amount of the sample;
ii. entrain the ejected aliquot in a gas or liquid stream; and
iii. transport the entrained aliquot into a mass spectrometer using the gas or liquid stream; and
d. the assaying system comprising the mass spectrometer and being configured to receive the entrained aliquot of the sample from the sample transfer system and to determine one or more characteristics of one or more eggs.

11. The system according to claim 10, wherein the acoustic transducer is configured to apply sound energy to at least an amount of the sample by controlling the energy.

12. The system according to any one of claims 9 to 11, wherein the sampling system comprises a means to determine the location of the air cell or allantois of the egg; and/or wherein the sampling system is configured to determine a sampling position based on one or more egg parameters, wherein the egg parameters are length of the egg, weight of the egg, color of the egg, image of the egg, dimension of the egg, circumference of the egg, incubation time of the egg, and/or age of the parent stock of the egg.

13. The system according to any one of claims 9 to 12, wherein the sample transfer system is configured to hold and transfer the sample via one or more instruments for holding comprising a multitude of sample containers and/or wherein the sample transfer system comprises a system that is configured to introduce the aliquot to a mass spectrometer comprising a sampling probe having an open end presenting an air interface for receiving the aliquot, an inlet connected to the sampling probe supplying a gas or liquid stream capable of entraining the aliquot, an outlet connected to the sampling probe receiving the gas or liquid stream, wherein the outlet is configured to be in fluid communication with the mass spectrometer.

14. The system according to any one of claims 9 to 13, wherein the characteristic is determined based on analyzing the amount of an analyte in the sample or an aliquot thereof wherein the analyte comprises at least one biomarker, preferably at least two biomarkers suitable for determining the characteristic, more preferably wherein at least one biomarker comprises a sex-related metabolite, such as a sex-related hormone, or a salt or a metabolite thereof, most preferably estron sulfate or 3-[(2-aminoethyl)sulfanyl]butanoic acid.

15. The system according to any one of claims 9 to 14, wherein the sampling system is in a fluid connection with the sample transfer system.

## Patentansprüche

1. Verfahren zum automatisierten Bestimmen einer Eigenschaft eines oder mehrerer Eier (104), die folgenden Schritte umfassend:
a. automatisiertes Entnehmen einer Probe aus einem oder mehreren Eiern;
i. Halten und Übertragen der Probe durch ein oder mehrere Instrumente an eine Einheit zum Halten einer oder mehrerer Proben;
ii. Auswerfen eines Aliquots aus der Probe durch Aufbringen von Schallenergie über einen Schallwandler oder von Lichtenergie auf mindestens eine Menge der Probe;
iii. Mitreißen des ausgeworfenen Aliquots in einem Gas- oder Flüssigkeitsstrom; und
iv. Transportieren des ausgeworfenen Aliquots zu einem Massenspektrometer unter Verwendung des Gas- oder Flüssigkeitsstroms;
und
b. automatisiertes Analysieren des Aliquots der Probe in dem Massenspektrometer auf das Fehlen oder Vorhandensein und die Menge eines oder mehrerer Moleküle, um eine oder mehrere Eigenschaften des Eies zu bestimmen.

2. Verfahren nach Anspruch 1, ferner umfassend, zu Schritt a.,
Bereitstellen eines Systems, das dazu konfiguriert ist, eine Probe oder ein Aliquot davon in ein Massenspektrometer einzuführen, wobei das System Folgendes umfasst
i. eine Probenahmesonde mit einem offenen Ende, das eine Luftschnittstelle zum Aufnehmen der Probe darstellt,
ii. einen Einlass, der mit der Probenahmesonde verbunden ist und einen Gas- oder Flüssigkeitsstrom liefert, der in der Lage ist, die Probe mitzureißen, und
iii. einen Auslass, der mit der Probenahmesonde verbunden ist und den Gas- oder Flüssigkeitsstrom aufnimmt, wobei der Auslass dazu konfiguriert ist, in Fluidkommunikation mit einem Massenspektrometer zu stehen.

3. Verfahren nach Anspruch 1 oder Anspruch 2, wobei das Aliquot ein durchschnittliches Volumen von mindestens 2 nl odervon 2 bis 10 nl aufweist.

4. Verfahren nach einem der Ansprüche 1 bis 3, wobei das Auswerfen des Aliquots umfasst, dass die Probe einen im Wesentlichen horizontalen oder konkaven Flüssigkeit-Luft-Schnittstellen-Oberflächenbereich aufweist.

5. Verfahren nach einem der Ansprüche 1 bis 4, wobei die Probe eine Allantoisflüssigkeitsprobe oder eine Blutprobe umfasst.

6. Verfahren nach einem der Ansprüche 1 bis 5, wobei der Gas- oder Flüssigkeitsstrom ein Lösungsmittel umfasst, wobei das Lösungsmittel Methanol umfasst und/oder wobei das eine oder die mehreren Moleküle 3-[(2-Aminoethyl)sulfanyl]butansäure umfassen.

7. Verfahren nach einem der Ansprüche 1 bis 6, wobei Schritt a. ferner Bestimmen des Standorts eines bevorzugten Probenentnahmepunkts auf der Eierschale und Sammeln der Probe an dem bevorzugten Probenentnahmepunkt umfasst, wobei der Standort des bevorzugten Probenentnahmepunkts auf der Eierschale einen Punkt auf der Schale des Eies auf einer Linie parallel zu der zentralen Eiachse umfasst, die einen Abstand von 0,5 bis 7 mm zu einem Schnittpunkt der Luftkammer mit der Allantois aufweist, wobei der Schnittpunkt innerhalb eines Abstands von 0 bis 2 mm von der Schale des Eies liegt, wobei der Abstand zu dem Schnittpunkt in der Richtung senkrecht zu der zentralen Eiachse gemessen wird.

8. Verfahren nach einem der Ansprüche 1 bis 7, wobei das Bestimmen der einen oder mehreren Eigenschaften des Eies Vergleichen der Menge des einen oder der mehreren Moleküle mit der Menge des einen oder der mehreren Moleküle umfasst, die in anderen Analysen nach dem Verfahren der Erfindung erhalten wurde.

9. System zur automatischen Bestimmung von Eieigenschaften, umfassend:
a. ein Fördersystem, das zum Transportieren eines oder mehrerer Eier (104) zu einem Probenahmesystem konfiguriert ist;
b. das Probenahmesystem, das dazu konfiguriert ist, eine Probe aus einem oder mehreren Eiern zu entnehmen, wobei das Probenahmesystem oder ein Teil davon zu einem Probenübertragungssystem bewegbar ist, oder umgekehrt, um die Probe zwischen dem Probenahmesystem und dem Probenübertragungssystem zu übertragen;
c. das Probenübertragungssystem, das dazu konfiguriert ist, die Probe zum Übertragen an ein Untersuchungssystem aufzunehmen, wobei das Probenübertragungssystem ein System umfasst, das dazu konfiguriert ist, die Probe zu übertragen, wobei das System, das dazu konfiguriert ist, die Probe zu übertragen, zu Folgendem konfiguriert ist:
i. Auswerfen eines Aliquots aus der Probe durch Aufbringen von Lichtenergie auf eine Menge der Probe;
ii. Mitreißen des ausgeworfenen Aliquots in einem Gas- oder Flüssigkeitsstrom; und
iii. Transportieren des mitgerissenen Aliquots in ein Massenspektrometer unter Verwendung des Gas- oder Flüssigkeitsstroms; und
d. das Untersuchungssystem, das das Massenspektrometer umfasst und dazu konfiguriert ist, das mitgerissene Aliquot der Probe von dem Probenübertragungssystem aufzunehmen und eine oder mehrere Eigenschaften eines oder mehrerer Eier zu bestimmen.

10. System zur automatischen Bestimmung von Eieigenschaften, umfassend:
a. ein Fördersystem, das zum Transportieren eines oder mehrerer Eier (104) zu einem Probenahmesystem konfiguriert ist;
b. das Probenahmesystem, das dazu konfiguriert ist, eine Probe aus einem oder mehreren Eiern zu entnehmen, wobei das Probenahmesystem oder ein Teil davon zu einem Probenübertragungssystem bewegbar ist, oder umgekehrt, um die Probe zwischen dem Probenahmesystem und dem Probenübertragungssystem zu übertragen;
c. das Probenübertragungssystem, das dazu konfiguriert ist, die Probe zum Übertragen an ein Untersuchungssystem aufzunehmen, wobei das Probenübertragungssystem einen Schallwandler und ein System umfasst, das dazu konfiguriert ist, die Probe zu übertragen, wobei das System, das dazu konfiguriert ist, die Probe zu übertragen, zu Folgendem konfiguriert ist:
i. Auswerfen eines Aliquots aus der Probe durch Aufbringen von Schallenergie über den Schallwandler auf eine Menge der Probe;
ii. Mitreißen des ausgeworfenen Aliquots in einem Gas- oder Flüssigkeitsstrom; und
iii. Transportieren des mitgerissenen Aliquots in ein Massenspektrometer unter Verwendung des Gas- oder Flüssigkeitsstroms; und
d. das Untersuchungssystem, das das Massenspektrometer umfasst und dazu konfiguriert ist, das mitgerissene Aliquot der Probe von dem Probenübertragungssystem aufzunehmen und eine oder mehrere Eigenschaften eines oder mehrerer Eier zu bestimmen.

11. System nach Anspruch 10, wobei der Schallwandler dazu konfiguriert ist, durch Steuern der Energie Schallenergie auf mindestens eine Menge der Probe aufzubringen.

12. System nach einem der Ansprüche 9 bis 11, wobei das Probenahmesystem ein Mittel zum Bestimmen des Standorts der Luftkammer oder der Allantois des Eies umfasst; und/oder wobei das Probenahmesystem dazu konfiguriert ist, eine Probenahmeposition basierend auf einem oder mehreren Eiparametern zu bestimmen, wobei die Eiparameter eine Länge des Eies, ein Gewicht des Eies, eine Farbe des Eies, ein Bild des Eies, eine Abmessung des Eies, ein Umfang des Eies, eine Bebrütungszeit des Eies und/oder ein Alter der Elterntiere des Eies sind.

13. System nach einem der Ansprüche 9 bis 12, wobei das Probenübertragungssystem dazu konfiguriert ist, die Probe über ein oder mehrere Instrumente zum Halten, die eine Vielzahl von Probenbehältern umfassen, zu halten und zu übertragen, und/oder wobei das Probenübertragungssystem ein System umfasst, das dazu konfiguriert ist, das Aliquot in ein Massenspektrometer einzuführen, und das eine Probenahmesonde mit einem offenen Ende, das eine Luftschnittstelle zum Aufnehmen des Aliquots darstellt, einen mit der Probenahmesonde verbundenen Einlass, der einen Gas- oder Flüssigkeitsstrom liefert, der in der Lage ist, das Aliquot mitzureißen, einen mit der Probenahmesonde verbundenen Auslass, der den Gas- oder Flüssigkeitsstrom aufnimmt, umfasst, wobei der Auslass dazu konfiguriert ist, in Fluidkommunikation mit dem Massenspektrometer zu stehen.

14. System nach einem der Ansprüche 9 bis 13, wobei die Eigenschaft basierend auf einem Analysieren der Menge eines Analyten in der Probe oder einem Aliquot davon bestimmt wird, wobei der Analyt mindestens einen Biomarker umfasst, vorzugsweise mindestens zwei Biomarker, die zum Bestimmen der Eigenschaft geeignet sind, wobei besonders bevorzugt mindestens ein Biomarker einen geschlechtsbezogenen Metaboliten umfasst, wie etwa ein geschlechtsbezogenes Hormon, oder ein Salz oder einen Metaboliten davon, am stärksten bevorzugt Östronsulfat oder 3-[(2-Aminoethyl)sulfanyl]butansäure.

15. System nach einem der Ansprüche 9 bis 14, wobei das Probenahmesystem in einer Fluidverbindung mit dem Probenübertragungssystem steht.

## Revendications

1. Procédé de détermination d'une caractéristique d'un ou de plusieurs oeufs (104) de manière automatisée, comprenant les étapes de :
a. extraction d'un échantillon d'un ou de plusieurs oeufs de manière automatisée ;
i. support et transfert de l'échantillon au moyen d'un ou plusieurs instruments vers une unité destinée à supporter un ou plusieurs échantillons ;
ii. éjection d'une aliquote de l'échantillon en appliquant de l'énergie sonore via un transducteur acoustique ou de l'énergie lumineuse à au moins une quantité de l'échantillon ;
iii. entraînement de l'aliquote éjectée dans un flux de gaz ou de liquide ; et
iv. transport de l'aliquote éjectée vers un spectromètre de masse en utilisant le flux de gaz ou de liquide ;
et
b. analyse de l'aliquote de l'échantillon de manière automatisée dans le spectromètre de masse pour détecter l'absence ou la présence et la quantité d'une ou plusieurs molécules, pour déterminer une ou plusieurs caractéristiques de l'œuf.

2. Procédé selon la revendication 1, comprenant en outre de l'étape a.,
la fourniture d'un système qui est configuré pour introduire un échantillon ou une aliquote de celui-ci dans un spectromètre de masse, le système comprenant
i. une sonde d'échantillonnage comportant une extrémité ouverte présentant une interface aérienne pour recevoir l'échantillon,
ii. une entrée reliée à la sonde d'échantillonnage fournissant un flux de gaz ou de liquide capable d'entraîner l'échantillon, et
iii. une sortie connectée à la sonde d'échantillonnage recevant le flux de gaz ou de liquide, dans lequel la sortie est configurée pour être en communication fluidique avec un spectromètre de masse.

3. Procédé selon la revendication 1 ou la revendication 2, dans lequel l'aliquote comporte un volume moyen d'au moins 2 nl ou de 2 à 10 nl.

4. Procédé selon l'une quelconque des revendications 1 à 3, dans lequel l'éjection de l'aliquote comprend l'échantillon comportant une surface d'interface liquide-air sensiblement horizontale ou concave.

5. Procédé selon l'une quelconque des revendications 1 à 4, dans lequel l'échantillon comprend un échantillon de liquide allantoïdien ou un échantillon de sang.

6. Procédé selon l'une quelconque des revendications 1 à 5, dans lequel le flux de gaz ou de liquide comprend un solvant, dans lequel le solvant comprend du méthanol et/ou dans lequel l'une ou plusieurs molécules comprennent de l'acide 3-[(2-aminoéthyl)sulfanyl]butanoïque.

7. Procédé selon l'une quelconque des revendications 1 à 6, dans lequel l'étape a. comprend en outre la détermination de l'emplacement d'un point d'extraction d'échantillon préféré sur la coquille d'oeuf et la collecte de l'échantillon au point d'extraction d'échantillon préféré, dans lequel l'emplacement du point d'extraction d'échantillon préféré sur la coquille d'oeuf comprend un point sur la coquille de l'œuf sur une ligne parallèle à l'axe central de l'œuf comportant une distance de 0,5 à 7 mm jusqu'à une intersection de l'alvéole avec l'allantoïde, l'intersection se trouvant à une distance de 0 à 2 mm de la coquille de l'œuf, la distance jusqu'à l'intersection étant mesurée dans la direction perpendiculaire à l'axe central de l'œuf.

8. Procédé selon l'une quelconque des revendications 1 à 7, dans lequel la détermination de l'une ou plusieurs caractéristiques de l'œuf comprend la comparaison de la quantité de l'une ou plusieurs molécules à la quantité de l'une ou plusieurs molécules obtenues dans d'autres analyses selon le procédé de l'invention.

9. Système de détermination automatisée de caractéristiques d'oeuf, comprenant :
a. un système de convoyeur configuré pour transporter un ou plusieurs oeufs (104) vers un système d'échantillonnage ;
b. le système d'échantillonnage étant configuré pour extraire un échantillon d'un ou de plusieurs oeufs, dans lequel le système d'échantillonnage ou une partie de celui-ci est mobile vers un système de transfert d'échantillon, ou vice versa, pour transférer l'échantillon entre le système d'échantillonnage et le système de transfert d'échantillon ;
c. le système de transfert d'échantillon étant configuré pour recevoir l'échantillon en vue de son transfert vers un système de test, dans lequel le système de transfert d'échantillon comprend un système qui est configuré pour transférer l'échantillon, dans lequel le système qui est configuré pour transférer l'échantillon est configuré pour :
i. éjecter une aliquote de l'échantillon en appliquant de l'énergie lumineuse à une quantité de l'échantillon ;
ii. entraîner l'aliquote éjectée dans un flux de gaz ou de liquide ; et
iii. transporter l'aliquote entraînée dans un spectromètre de masse en utilisant le flux de gaz ou de liquide ; et
d. le système de test comprenant le spectromètre de masse et étant configuré pour recevoir l'aliquote entraînée de l'échantillon en provenance du système de transfert d'échantillon et pour déterminer une ou plusieurs caractéristiques d'un ou de plusieurs oeufs.

10. Système de détermination automatisée de caractéristiques d'oeuf, comprenant :
a. un système de convoyeur configuré pour transporter un ou plusieurs oeufs (104) vers un système d'échantillonnage ;
b. le système d'échantillonnage étant configuré pour extraire un échantillon d'un ou de plusieurs oeufs, dans lequel le système d'échantillonnage ou une partie de celui-ci est mobile vers un système de transfert d'échantillon, ou vice versa, pour transférer l'échantillon entre le système d'échantillonnage et le système de transfert d'échantillon ;
c. le système de transfert d'échantillon étant configuré pour recevoir l'échantillon à transférer vers un système de test, le système de transfert d'échantillon comprenant un transducteur acoustique et un système qui est configuré pour transférer l'échantillon, dans lequel le système qui est configuré pour transférer l'échantillon est configuré pour :
i. éjecter une aliquote de l'échantillon en appliquant de l'énergie sonore via le transducteur acoustique à une quantité de l'échantillon ;
ii. entraîner l'aliquote éjectée dans un flux de gaz ou de liquide ; et
iii. transporter l'aliquote entraînée dans un spectromètre de masse en utilisant le flux de gaz ou de liquide ; et
d. le système de test comprenant le spectromètre de masse et étant configuré pour recevoir l'aliquote entraînée de l'échantillon en provenance du système de transfert d'échantillon et pour déterminer une ou plusieurs caractéristiques d'un ou de plusieurs oeufs.

11. Système selon la revendication 10, dans lequel le transducteur acoustique est configuré pour appliquer de l'énergie sonore à au moins une quantité de l'échantillon en commandant l'énergie.

12. Système selon l'une quelconque des revendications 9 à 11, dans lequel le système d'échantillonnage comprend un moyen pour déterminer l'emplacement de l'alvéole ou de l'allantoïde de l'œuf ; et/ou dans lequel le système d'échantillonnage est configuré pour déterminer une position d'échantillonnage sur la base d'un ou plusieurs paramètres de l'œuf, les paramètres de l'œuf étant la longueur de l'œuf, le poids de l'œuf, la couleur de l'œuf, l'image de l'œuf, la dimension de l'œuf, la circonférence de l'œuf, le temps d'incubation de l'œuf et/ou l'âge du reproducteur de l'œuf.

13. Système selon l'une quelconque des revendications 9 à 12, dans lequel le système de transfert d'échantillon est configuré pour supporter et transférer l'échantillon via un ou plusieurs instruments de support comprenant une multitude de contenants d'échantillon et/ou dans lequel le système de transfert d'échantillon comprend un système qui est configuré pour introduire l'aliquote dans un spectromètre de masse comprenant une sonde d'échantillonnage comportant une extrémité ouverte présentant une interface d'air pour recevoir l'aliquote, une entrée connectée à la sonde d'échantillonnage fournissant un flux de gaz ou de liquide capable d'entraîner l'aliquote, une sortie connectée à la sonde d'échantillonnage recevant le flux de gaz ou de liquide, dans lequel la sortie est configurée pour être en communication fluidique avec le spectromètre de masse.

14. Système selon l'une quelconque des revendications 9 à 13, dans lequel la caractéristique est déterminée sur la base de l'analyse de la quantité d'un analyte dans l'échantillon ou une aliquote de celui-ci, dans lequel l'analyte comprend au moins un biomarqueur, de préférence au moins deux biomarqueurs appropriés pour déterminer la caractéristique, plus préférablement dans lequel au moins un biomarqueur comprend un métabolite lié au sexe, tel qu'une hormone liée au sexe, ou un sel ou un métabolite de celui-ci, le plus préférablement le sulfate d'estrone ou l'acide 3-[(2-aminoéthyl)sulfanyl]butanoïque.

15. Système selon l'une quelconque des revendications 9 à 14, dans lequel le système d'échantillonnage est en connexion fluidique avec le système de transfert d'échantillon.
